# EUROPEAN PATENT APPLICATION

(11) **EP 1 354 587 A1**
(43) Date of publication of application: **22.10.2003**
(21) Application number: 01271854.0
(22) Date of filing: 21.12.2001
(51) Int. Cl.: A61K 31/445, A61K 9/107, C07D 211/62

(54) **MEDICINAL COMPOSITIONS FOR ORAL USE**

(30) Priority: 22.12.2000 JP 2000390870
(71) Applicant: Takeda Chemical Industries, Ltd., Osaka-shi, Osaka 541-0045 (JP)
(72) Inventor: AKIYAMA, Yohko, Omihachiman-shi, Shiga 523-0898 (JP); NAGAHARA, Naoki, Kawabe-gun, Hyogo 666-0257 (JP)
(74) Representative: Rickard, Timothy Mark Adrian
(86) International application number: JP0111232
(87) International publication number: WO02051414

(57) **Abstract**

An oral pharmaceutical composition of the present invention, which contains a compound having CCR5 antagonistic action dissolved or dispersed in an amphiphilic substance-containing carrier, shows less dispersion in the blood concentration of a compound having CCR5 antagonistic action or a salt thereof, shows extremely fine oral absorption, and makes a preparation for oral administration useful for the prophylaxis or treatment of various HIV infectious diseases in human.

## Description

### Technical Field

The present invention relates to an oral pharmaceutical composition comprising a compound having CCR5 antagonistic action or a salt thereof.

### Background Art

In addition to CD4, which has been conventionally known as a receptor in invasion of HIV (human immunodeficiency virus) into a target cell, CCR5, which is a G-protein-coupled chemokine receptor having seven transmembrane domains, has been recently discovered as a second receptor of macrophage-tropic HIV. It is reported that a person who is resistant to HIV infection in spite of several exposures retains mutation of homo deletion of CCR5 gene. Therefore, a CCR5 antagonist is expected to be a new anti-HIV drug.

A newly synthesized compound having a piperidine skeleton has a superior HIV inhibitory activity and its development as an orally administrable novel anti-HIV drug is expected. The possibility of development of an oral preparation depends on the bioavailability of the drug. When the bioavailability is low, it causes inconsistent effects in humans, making the development difficult. As factors affecting the bioavailability of the drug, drug solubility, interaction with gastrointestinal mucosal layer, gastrointestinal mucosal permeability, first-pass effect and the like are recited. A piperidine compound having CCR5 antagonistic action has, like the conventional anti-HIV drugs, a defect of low bioavailability by oral administration. It is therefore important to improve oral absorption of such compound or a salt thereof for stable expression of the efficacy of a CCR5 antagonist.

As mentioned above, a CCR5 antagonist compound having a piperidine skeleton is most desirably developed as an oral preparation in view of compliance. However, since the low bioavailability of a preparation causes inconsistent therapeutic effects, the development of an oral preparation containing a piperidine compound having CCR5 antagonistic action with promoted oral absorption or improved bioavailability or a salt thereof is currently desired.

### Disclosure of the Invention

In view of such situation, the present inventors have considered the formulation of a novel oral preparation superior in absorption and having high bioavailability and found that, by dissolving or microdispersing a piperidine compound having CCR5 antagonistic action or a salt thereof in a certain kind of substance, oral absorption can be promoted, based on which finding the present invention has been completed.

Accordingly, the present invention relates to
(1) an oral pharmaceutical composition comprising a compound having CCR5 antagonistic action, which is a compound represented by the formula 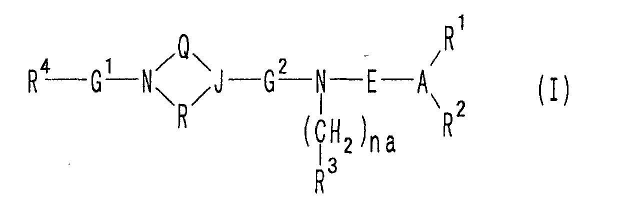 wherein
   - R¹: is a hydrogen atom, a hydrocarbon group optionally having substituent(s) or a nonaromatic heterocyclic group optionally having substituent(s),
   - R²: is a hydrocarbon group optionally having substituent(s), a nonaromatic heterocyclic group optionally having substituent(s),
   alternatively R¹ and R² may combine to form, together with A, a heterocyclic group optionally having substituent(s),
   - A: is N or N+-R⁵·Y⁻ (R⁵ is a hydrocarbon group and Y⁻ is a counter anion),
   - R³: is a cyclic hydrocarbon group optionally having substituent(s) or a heterocyclic group optionally having substituent(s),
   - na: is 0 or 1,
   - R⁴: is a hydrogen atom, a hydrocarbon group optionally having substituent(s), a heterocyclic group optionally having substituent(s), an alkoxy group optionally having substituent(s), an aryloxy group optionally having substituent(s) or an amino group optionally having substituent(s),
   - E: is a divalent chain hydrocarbon group optionally having substituent(s) other than oxo group,
   - G₁: is a bond, CO or SO₂,
   - G²: is CO, SO₂, NHCO, CONH or OCO,
   - J: is a methine or a nitrogen atom, and
   - Q and R: are each a bond or a divalent C₁₋₃ chain hydrocarbon optionally having substituent(s),
   provided that when G² is OCO, J is a methine, and neither Q nor R is a bond, and when G¹ is a bond, neither Q nor R is substituted by oxo group,
   or a salt thereof, or a compound represented by the formula 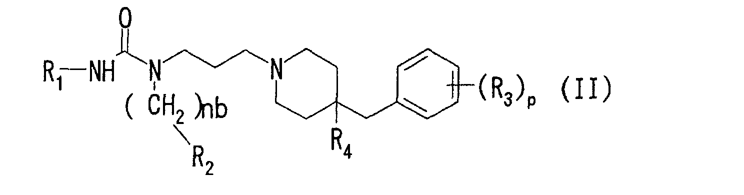 wherein
   - R₁: is a hydrocarbon group optionally having substituent(s),
   - R₂: is a cyclic hydrocarbon group optionally having substituent(s) or a heterocyclic group optionally having substituent(s),
   - R₃: is a halogen atom, a carbamoyl group optionally having substituent(s), a sulfamoyl group optionally having substituent(s), an acyl group derived from sulfonic acid, a C₁₋₄ alkyl group optionally having substituent(s), a C₁₋₄ alkoxy group optionally having substituent(s), an amino group optionally having substituent(s), a nitro group or a cyano group,
   - R₄: is a hydrogen atom or a hydroxy group,
   - nb: is 0 or 1, and
   - p: is 0 or an integer of 1 to 4, or a salt thereof,
   which is dissolved or dispersed in at least one amphiphilic substance;
(2) the composition of the above-mentioned (1), wherein R¹ is a hydrogen atom, a hydrocarbon group selected from the following Group 2, which optionally has substituent(s) selected from the following Group 1, a 3- to 8-membered saturated or unsaturated nonaromatic heterocyclic group optionally having substituent(s) selected from the following Group 1, R² is a hydrocarbon group selected from the following Group 2, which optionally has substituent(s) selected from the following Group 1 or a 3- to 8-membered saturated or unsaturated nonaromatic heterocyclic group optionally having substituent(s) selected from the following Group 1, alternatively R¹ and R² may combine to form, together with A, a heterocyclic group selected from the following Group 4, which optionally has substituent(s) selected from the following Group 3, A is N or N⁺-R5·Y⁻ (Y⁻ is Cl⁻ Br⁻, I⁻, NO₃⁻, SO₄²⁻, PO₄³⁻ or CH₃SO₃⁻, and R⁵ is a hydrocarbon group selected from the following Group 2), R³ is a cyclic hydrocarbon group selected from the following Group 5, which optionally has substituent(s) selected from the following Group 1 or a heterocyclic group selected from the following Group 6, which optionally has substituent(s) selected from the following Group 1, R⁴ is a hydrogen atom, a hydrocarbon group selected from the following Group 2, which optionally has substituent(s) selected from the following Group 1, a heterocyclic group selected from the following Group 6, which optionally has substituent(s) selected from the following Group 1, a C₁₋₆ alkoxy group optionally having substituent(s) selected from the following Group 7, a C₆₋₁₄ aryloxy group optionally having substituent(s) selected from the following Group 8, an amino group optionally having substituent(s) selected from the following Group 9 or a cyclic amino group selected from the following Group 10, E is a divalent chain hydrocarbon group selected from the following Group 12, which optionally has substituent(s) other than oxo group selected from the following Group 12, Q and R are each a bond or a divalent C₁₋₃ chain hydrocarbon group selected from the following Group 13, which optionally has substituent(s) selected from the following Group 11, R₁ is a hydrocarbon group selected from the following Group 31, which optionally has substituent(s) selected from Group 29, R₂ is a cyclic hydrocarbon group selected from the following Group 35, which optionally has substituent(s) selected from Group 30 or a heterocyclic group selected from Group 32, which optionally has substituent(s) selected from Group 30, and R₃ is a halogen atom, a carbamoyl group, an N-monosubstituted carbamoyl group optionally having one selected from Group 19, an N,N-disubstituted carbamoyl group optionally having one selected from Group 19 and one selected from Group 20, a cyclic aminocarbonyl group selected from Group 21, sulfamoyl group, an N-monosubstituted sulfamoyl group optionally having one selected from Group 19, an N,N-disubstituted sulfamoyl group optionally having one selected from Group 19 and one selected from Group 20, a cyclic aminosulfonyl group selected from Group 36, an acyl group selected from Group 22, which is derived from sulfonic acid, a C₁₋₄ alkyl group optionally having substituent(s) selected from Group 30, a C₁₋₄ alkoxy group optionally having substituent(s) selected from Group 30, an amino group optionally having substituent(s) selected from Group 33, a cyclic amino group selected from Group 34, a nitro group or a cyano group, wherein in the above-mentioned,
   Group 1 includes
   1) a C₁₋₆ alkyl group optionally substituted by a group selected from Group 14, 2) a C₂₋₆ alkenyl group optionally substituted by a group selected from Group 14, 3) a C₂₋₆ alkynyl group optionally substituted by a group selected from Group 14, 4) a C₆₋₁₄ aryl group optionally substituted by a group selected from Group 14, 5) a C₃₋₇ cycloalkyl group optionally substituted by a group selected from Group 14, 6) a C₃₋₆ cycloalkenyl group optionally substituted by a group selected from Group 14, 7) a heterocyclic group selected from Group 16, which is optionally substituted by a group selected from Group 15, 8)an amino group optionally having, as a substituent, C₁₋₆ alkylimidoyl, formylimidoyl, amidino or a group selected from Group 17, 9) a cyclic amino group selected from Group 10, 10) an imidoyl group optionally substituted by a group selected from Group 17, 11) an amidino group optionally substituted by a group selected from Group 17, 12) a hydroxy group optionally substituted by a group selected from Group 17, 13) a thiol group optionally substituted by a group selected from Group 17, 14) a carboxyl group, 15) a C₁₋₆ alkoxy-carbonyl group optionally substituted by a group selected from Group 18, 16) a C₇₋₁₂ aryloxy-carbonyl group optionally substituted by a group selected from Group 18, 17) a C₇₋₁₀ aralkyloxy-carbonyl group optionally substituted by a group selected from Group 18, 18) a carbamoyl group, 19) a monosubstituted carbamoyl group substituted by a group selected from Group 19, 20) a disubstituted carbamoyl group substituted by one selected from Group 19 and one selected from Group 20, 21) a cyclic aminocarbonyl group selected from Group 21, 22) a thiocarbamoyl group, 23) a monosubstituted thiocarbamoyl group substituted by a group selected from Group 19, 24) a disubstituted thiocarbamoyl group substituted by one selected from Group 19 and one selected from Group 20, 25) sulfamoyl group, 26) an N-monosubstituted sulfamoyl group substituted by a group selected from Group 19, 27) an N,N-disubstituted sulfamoyl group substituted by one selected from Group 19 and one selected from Group 20, 28) a cyclic aminosulfonyl group selected from Group 22, 29) a halogen atom, 30) a cyano group, 31) a nitro group, 32) an acyl group selected from Group 22, which is derived from sulfonic acid, 33) a formyl group, 34) a C₂₋₆ alkanoyl, 35) a C₇₋₁₂ arylcarbonyl, 36) a C₁₋₆ alkylsulfinyl group optionally substituted by a group selected from Group 23 and 37) a C₆₋₁₄ arylsulfinyl group optionally substituted by a group selected from Group 23,
      Group 2 includes

   1) a C₁₋₁₀ alkyl group, 2) a C₂₋₆ alkenyl group, 3) a C₂₋₆ alkynyl group, 4) a C₃₋₉ cycloalkyl group optionally condensed with benzene ring(s), 5) a C₃₋₆ cycloalkenyl group, 6) a C₄₋₆ cycloalkanedienyl group and 7) a C₆₋₁₄ aryl group,
      Group 3 includes

   1) a hydroxy group, 2) a cyano group, 3) a nitro group, 4) an amino group, 5) an oxo group, 6) a halogen atom and 7) a group represented by the general formula -B¹R^{a} wherein R^{a} is a hydrocarbon group selected from Group 2 optionally having substituent(s) selected from Group 1, or a heterocyclic group selected from Group 6 optionally having substituent(s) selected from Group 1, and B¹ is a bond (single bond), -CR^{b}R^{c}-, -COO-, -CO-, -CR^{b}(OH)-, -CR^{b}R^{c}-S-, -CR^{b}R^{c}-SO₂-, -CO-NR^{b}-, -CS-NR^{b}-,-CO-S-,-CS-S-, -CO-NR^{b}-CO-NR^{c}-, -C(=NH)-NR^{b}-, -NR^{b}-, -NR^{b}-CO-, -NR^{b}-CS-, -NR^{b}-CO-NR^{c}-, -NR^{b}-CS-NR^{c}-, -NR^{b}-CO-O-, -NR^{b}-CS-O-, -NR^{b}-CO-S-, -NR^{b}-CS-S-, -NR^{b}-C(=NH)-NR^{c}-, -NR^{b}SO₂-, -NR^{b}NR^{c}-, -O-, -O-CO-, -O-CS-, -O-CO-O-, -O-CO-NR^{b}-, -O-C (=NH)-NR^{b}-, -S-, -SO-, -SO₂-, -SO₂-NR^{b}-, -S-CO-, -S-CS-, -S-CO-NR^{b}-, -S-CS-NR^{b}- and -S-C(=NH)-NR^{b}- (wherein R^{b} and R^{c} are each a hydrogen atom, a C₁₋₆ alkyl group optionally substituted by a group selected from Group 14, a C₂₋₆ alkenyl group optionally substituted by a group selected from Group 14, a C₂₋₆ alkynyl group optionally substituted by a group selected from Group 14, a C₆₋₁₄ aryl group optionally substituted by a group selected from Group 14, a C₃₋₇ cycloalkyl group optionally substituted by a group selected from Group 14, a C₃₋₆ cycloalkenyl group optionally substituted by a group selected from Group 14, a heterocyclic group selected from Group 6, which is optionally substituted by a group selected from Group 1, an acyl group selected from Group 22, which is derived from sulfonic acid, a C₁₋₆ alkanoyl or a C₇₋₁₂ arylcarbonyl group),
      Group 4 includes

   1) a monocyclic heterocyclic group, 2) a fused heterocyclic ring wherein benzene is condensed and 3) a heterospirocyclic ring, each of which may further contain, besides one nitrogen atom, nitrogen atom, oxygen atom, sulfur atom,
      Group 5 includes

   1) a C₃₋₉ cycloalkyl optionally condensed with benzene ring(s),
   2) a C₃₋₆ cycloalkenyl group, 3) a C₄₋₆ cycloalkanedienyl group and 4) a C₆₋₁₄ aryl group,
      Group 6 includes

   1) a 5- or 6-membered aromatic monocyclic heterocyclic group selected from Group 24, 2) a 8- to 12-membered aromatic fused heterocycle group selected from Group 26 and 3) a 3- to 8-membered saturated or unsaturated nonaromatic heterocyclic group selected from Group 25 (aliphatic heterocyclic group), which heterocyclic groups contain, as a ring constituting atom (ring atom), 1 to 3 (at least 1) kinds of hetero atoms selected from oxygen atom, sulfur atom and nitrogen atom,
      Group 7 includes
      a C₃₋₆ cycloalkyl group optionally substituted by a group selected from Group 18, a C₆₋₁₀ aryl group optionally substituted by a group selected from Group 18, a C₇₋₁₀ aralkyl group optionally substituted by a group selected from Group 18 and a heterocyclic group selected from Group 16, which is optionally substituted by a group selected from Group 18,
      Group 8 includes
      a C₁₋₆ alkoxy group, a halogen atom, a C₁₋₆ alkyl group, an amino group, a hydroxy group, a cyano group and an amidino group,
      Group 9 includes

   1) a C₁₋₆ alkyl group, 2) a C₁₋₆ alkanoyl, 3) benzoyl, 4) an optionally halogenated C₁₋₆ alkoxy-carbonyl, 5) a C₁₋₆ alkylimidoyl, 6) formylimidoyl and 7) amidino,
      Group 10 includes

   1) 1-azetidinyl, 2) 1-pyrrolidinyl, 3) 1-piperidinyl, 4) 4-morpholinyl and 5) 1-piperazinyl optionally having substituent(s) selected from Group 27,
      Group 11 includes

   1) a C₁₋₆ alkyl group optionally substituted by a group selected from Group 14, 2) a C₆₋₁₄ aryl group optionally substituted by a group selected from Group 14, 3) a C₃₋₇ cycloalkyl group optionally substituted by a group selected from Group 14, 4) a C₃₋₆ cycloalkenyl group optionally substituted by a group selected from Group 14, 5) a carboxyl group, 6) a C₁₋₆ alkoxy-carbonyl group optionally substituted by a group selected from Group 18, 7) a C₇₋₁₂ aryloxy-carbonyl group optionally substituted by a group selected from Group 18, 8) a C₇₋₁₀ aralkyloxy-carbonyl group optionally substituted by a group selected from Group 18, 9) a carbamoyl group, 10) a monosubstituted carbamoyl group substituted by a group selected from Group 19, 11) a disubstituted carbamoyl group substituted by one selected from Group 19 and one selected from Group 20, 12) a cyclic aminocarbonyl group selected from Group 21, 13) a thiocarbamoyl group, 14) a monosubstituted thiocarbamoyl group substituted by a group selected from Group 19, 15) a disubstituted thiocarbamoyl group substituted by one selected from Group 19 and one selected from Group 20, 16) an amino group optionally having, as a substituent, C₁₋₆ alkylimidoyl, formylimidoyl, amidino and a group selected from Group 17, 17) a cyclic amino group selected from Group 10, 18) a hydroxy group optionally substituted by a group selected from Group 17, 19) a thiol group optionally substituted by a group selected from Group 17, 20) C₁₋₆ alkanoyl, 21) C₇₋₁₂ arylcarbonyl, 22) an acyl group selected from Group 22, which is derived from sulfonic acid, 23) halogen, 24) nitro and 25) cyano,
      Group 12 includes
      a C₁₋₆ alkylene, a C₂₋₆ alkenylene and a C₂₋₆ alkynylene,
      Group 13 includes
      a C₁₋₃ alkylene, a C₂₋₃ alkenylene and a C₂₋₃ alkynylene,
      Group 14 includes

   1) a C₁₋₆ alkoxy group optionally substituted by halogen, 2) a phenoxy optionally having halogen or carbamoyl as a substituent, 3) a halogen atom, 4) a C₁₋₆ alkyl group, 5) a halogen-substituted C₁₋₄ alkyl group, 6) a C₃₋₈ cycloalkyl, 7) an amino group, 8) an amino group having, as a substituent, 1 or 2 of carbamoyl, C₁₋₄ alkyl and C₁₋₄ alkylsulfonyl, 9) a carbamoyl group optionally substituted by C₁₋₆ alkyl, 10) a formyl, 11) a C₂₋₆ alkanoyl group, 12) a C₆₋₁₄ aryl group, 13) a C₆₋₁₄ arylcarbonyl, 14) a C₇₋₁₃ aralkylcarbonyl, 15) a hydroxy group, 16) a C₂₋₅ alkanoyloxy, 17) a C₇₋₁₃ aralkylcarbonyloxy, 18) a nitro group, 19) a sulfamoyl group, 20) an N-C₁₋₄ alkylsulfamoyl, 21) phenylthio, 22) a C₁₋₄ alkylphenylthio, 23) -N=N-phenyl, 24) a cyano group, 25) an oxo group, 26) an amidino group, 27) a carboxyl group, 28) a C₁₋₄ alkoxycarbonyl, 29) a C₁₋₆ alkylthio, 30) a C₁₋₆ alkylsulfinyl, 31) a C₁₋₆ alkylsulfonyl, 32) a C₆₋₁₄ arylthio, 33) a C₆₋₁₄ arylsulfinyl, 34) a C₆₋₁₄ arylsulfonyl and 35) a heterocyclic group selected from Group 6,
      Group 15 includes
      a C₁₋₆ alkyl group, a C₁₋₆ alkanoyl, a C₇₋₁₃ arylcarbonyl, a C₁₋₆ alkylsulfonyl, aminosulfonyl, a mono-C₁₋₆ alkylaminosulfonyl, a di-C₁₋₆ alkylaminosulfonyl and a C₁₋₄ alkyl halide,
      Group 16 includes

   1) an aromatic heterocyclic group selected from Group 24 and Group 26 and 2) a saturated or unsaturated nonaromatic heterocyclic group selected from Group 25, which heterocyclic groups containing, as a ring constituting atom (ring atom), 1 to 3 (at least 1) kinds of hetero atoms selected from oxygen atom, sulfur atom and nitrogen atom,
      Group 17 includes

   1) a C₁₋₆ alkyl group optionally having halogen or C₁₋₆ alkoxy as a substituent, 2) a C₆₋₁₂ aryl group, 3) a C₁₋₄ alkyl-substituted C₆₋₁₂ aryl group, 4) a C₃₋₈ cycloalkyl group optionally having halogen or C₁₋₆ alkoxy as a substituent, 5) a C₁₋₆ alkoxy group, 6) a C₁₋₆ alkanoyl, 7) a C₇₋₁₃ arylcarbonyl, 8) a C₁₋₄ alkyl-substituted C₇₋₁₃ arylcarbonyl, 9) a C₁₋₆ alkylsulfonyl, 10) a C₆₋₁₄ arylsulfonyl, 11) aminosulfonyl, 12) a substituted aminosulfonyl mono- or di-substituted by C₁₋₄ alkyl and 13) an optionally halogenated C₁₋₆ alkoxy-carbonyl,
   Group 18 includes
   1) a hydroxy group, 2) an amino group, 3) an amino group mono- or di-substituted by a group selected from Group 28, 4) a halogen atom, 5) a nitro group, 6) a cyano group, 7) a C₁₋₆ alkyl group optionally substituted by halogen atom and 8) a C₁₋-₆ alkoxy group optionally substituted by halogen atom,
   Group 19 includes
   a C₁₋₆ alkyl group optionally substituted by a group selected from Group 18, a C₃₋₆ cycloalkyl group optionally substituted by a group selected from Group 18, a C₆₋₁₀ aryl group optionally substituted by a group selected from Group 18, a C₇₋₁₀ aralkyl group optionally substituted by a group selected from Group 18, a C₁₋₆ alkoxy group optionally substituted by a group selected from Group 18 and a heterocyclic group selected from Group 16, which is optionally substituted by a group selected from Group 18,
   Group 20 includes
   a C₁₋₆ alkyl group, a C₃₋₆ cycloalkyl group and a C₇₋₁₀ aralkyl group,
   Group 21 includes
   1-azetidinylcarbonyl, 1-pyrrolidinylcarbonyl, 1-piperidinylcarbonyl, 4-morpholinylcarbonyl and 1-piperazinylcarbonyl optionally substituted by a group selected from Group 27,
   Group 22 includes
   a C₁₋₁₀ alkylsulfonyl optionally having substituent(s) selected from Group 18, a C₂₋₆ alkenylsulfonyl optionally having substituent(s) selected from Group 18, a C₂₋₆ alkynylsulfonyl optionally having substituent(s) selected from Group 18, a C₃₋₉ cycloalkylsulfonyl optionally having substituent(s) selected from Group 18, a C₃₋₉ cycloalkenylsulfonyl optionally having substituent(s) selected from Group 18, a C₆₋₁₄ arylsulfonyl optionally having substituent(s) selected from Group 18 and a C₇₋₁₀ aralkylsulfonyl optionally having substituent(s) selected from Group 18,
   Group 23 includes
   a C₁₋₆ alkoxy group, a halogen atom, a C₁₋₆ alkyl group, an amino group, a hydroxy group, a cyano group and an amidino group,
   Group 24 includes
   furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl,
   Group 25 includes
   oxyranyl, azetidinyl, oxetanyl, thietanyl, pyrrolidinyl, tetrahydrofuryl, thiolanyl, piperidinyl, tetrahydropyranyl, morpholinyl, thiomorpholinyl and piperazinyl,
   Group 26 includes
   benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, benzodioxolyl, benzimidazolyl, 2,1,1-benzoxadiazolyl, 1H-benzotriazolyl, quinolyl, isoquinolyl, cinnolinyl, quinazolynyl, quinoxalinyl, phthalazinyl, naphthylidinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl,
   Group 27 includes
   a C₁₋₆ alkyl group, a C₇₋₁₀ aralkyl group and a C₆₋₁₀ aryl group,
   Group 28 includes
   a C₁₋₆ alkyl group, a C₁₋₆ alkanoyl, a C₇₋₁₃ arylcarbonyl and a C₁₋₆ alkylsulfonyl,
   Group 29 includes
   1) a hydrocarbon group selected from Group 31, which optionally has substituent(s) selected from Group 30, 2) a heterocyclic group selected from Group 32, which optionally has substituent(s) selected from Group 30, 3) a C₁₋₄ alkoxy group optionally having substituent(s) selected from Group 30, 4) a C₁₋₄ alkylthio group optionally having substituent(s) selected from Group 30, 5) a C₂₋₆ alkoxycarbonyl group optionally having substituent(s) selected from Group 30, 6) a C₁₋₆ alkanoyl group, 7) an amino group optionally having substituent(s) selected from Group 33, 8) a cyclic amino group selected from Group 34, 9) a halogen atom, 10) a nitro group, 11) a cyano group, 12) a carbamoyl group, 13) a monosubstituted carbamoyl group substituted by a group selected from Group 19, 14) a disubstituted carbamoyl group substituted by one selected from Group 19 and one selected from Group 20, 15) a cyclic aminocarbonyl group selected from Group 21, 16) a sulfamoyl group, 17) an N-monosubstituted sulfamoyl group substituted by a group selected from Group 19, 18) an N,N-disubstituted sulfamoyl group substituted by one selected from Group 19 and one selected from Group 20, 19) an acyl group selected from Group 22, which is derived from sulfonic acid,
   Group 30 includes
   1) a C₁₋₆ alkoxy group, 2) a halogen atom, 3) a C₁₋₆ alkyl group, 4) a C₁₋₄ alkynyl group, 5) an amino group, 6) a hydroxy group, 7) a cyano group and 8) an amidino group,
   Group 31 includes
   1) a C₁₋₆ alkyl group, 2) a C₃₋₈ cycloalkyl group and 3) a C₆₋₁₄ aryl group,
   Group 32 includes
   1) an aromatic monocyclic heterocyclic group selected from Group 24, 2) an aromatic fused heterocyclic group selected from Group 26 and 3) a saturated or unsaturated nonaromatic heterocyclic group selected from Group 25,
   Group 33 includes
   1) a C₁₋₆ alkyl, 2) a C₁₋₆ alkanoyl, 3) a C₇₋₁₃ arylcarbonyl, 4) an optionally halogenated C₂₋₆ alkoxycarbonyl, 5) a C₁₋₆ alkylimidoyl, 6) a formylimidoyl and 7) an amidino,
   Group 34 includes
   1) 1-azetidinyl, 2) 1-pyrrolidinyl, 3) 1-piperidinyl, 4) 4-morpholinyl, 5) 1-piperazinyl and 6) a 1-piperazinyl optionally having C₁₋₆ alkyl, C₇₋₁₀ aralkyl or C₆₋₁₀ aryl at the 4-position,
   Group 35 includes
   a C₃₋₉ cycloalkyl, 1-indanyl, 2-indanyl, a C₃₋₆ cycloalkenyl, a C₄₋₆ cycloalkanedienyl and a C₆₋₁₄ aryl,
   Group 36 includes
   1-azetidinylsulfonyl, 1-pyrrolidinylsulfonyl, 1-piperidinylsulfonyl, 4-morpholinylsulfonyl and a 1-piperazinylsulfonyl optionally substituted by a group selected from Group 27;
(3) the composition of the above-mentioned (1), wherein the compound having CCR5 antagonistic action of the above-mentioned (1) is dissolved in at least one amphiphilic substance;
(4) the composition of the above-mentioned (1), wherein the amphiphilic substance contains at least one solubilizing agent;
(5) the composition of the above-mentioned (1), wherein the amphiphilic substance contains at least one kind of lipid;
(6) the composition of the above-mentioned (1), which is a liquid or semi-solid at about 15°C to about 25°C;
(7) the composition of the above-mentioned (1), which is solid at about 15°C to about 25°C;
(8) the composition of the above-mentioned (1), which has a self-emulsifying action;
(9) the composition of the above-mentioned (1), wherein the amphiphilic substance comprises a combination of one or more kinds selected from water-soluble vitamin E derivatives, saturated polyglycolated glycerides, unsaturated polyglycolated glycerides, sorbitan fatty acid esters, polyglycerine fatty acid esters, polyoxyethylene polypropylene glycols, polyoxyethylene castor oil derivatives and propylene glycol laurates;
(10) the composition of the above-mentioned (1), wherein the amphiphilic substance comprises a combination of one or more kinds selected from water-soluble vitamin E derivatives, saturated polyglycolated glycerides, unsaturated polyglycolated glycerides;
(11) the composition of the above-mentioned (1), comprising a water-soluble vitamin E derivative as an amphiphilic substance;
(12) the composition of the above-mentioned (11), wherein the water-soluble vitamin E derivative is D-α-tocopherol polyethylene glycol 1000 succinate;
(13) the composition of the above-mentioned (1), wherein the emulsion is formed by adding water to a system wherein a compound having CCR5 antagonistic action is dissolved or dispersed in at least one amphiphilic substance;
(14) the composition of the above-mentioned (1), wherein the compound having CCR5 antagonistic action is N-(3,4-dichlorophenyl)-1-(methylsulfonyl)-N-{3-[4-({4-[(methylsulfonyl)amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl)-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide or a salt thereof;
(15) an oral pharmaceutical composition wherein a piperidine compound having CCR5 antagonistic action is dissolved or dispersed in at least one amphiphilic substance;
(16) the composition of the above-mentioned (1), comprising the piperidine compound having CCR5 antagonistic action of the above-mentioned (1), a protease inhibitor and/or a reverse transcriptase inhibitor in combination;
(17) a capsule containing the composition of the above-mentioned (1);
(18) the composition of the above-mentioned (1), comprising water;
(19) a process for preparing an oral pharmaceutical composition, which comprises preparing the same from an aqueous dispersion or aqueous solution of a piperidine compound having CCR5 antagonistic action and at least one amphiphilic substance;
(20) the process of the above-mentioned (19), comprising adding a piperidine compound having CCR5 antagonistic action to an aqueous solution containing at least one amphiphilic substance and stirring;
(21) a process for preparing an oral pharmaceutical composition, which comprises dissolving or dispersing a piperidine compound having CCR5 antagonistic action in an amphiphilic substance melted at a temperature higher than the melting point of the amphiphilic substance in a homogenous ' micro-state, followed by cooling;
(22) the composition of the above-mentioned (1), which is a prophylactic or therapeutic agent of an HIV infectious disease;
(23) the composition of the above-mentioned (1), which is a prophylactic or therapeutic agent of AIDS;
(24) a method of prophylaxis or treatment of HIV infection, which comprises administration of the oral pharmaceutical composition of the above-mentioned (1) to a mammal;
(25) use of at least one amphiphilic substance for the production of the oral pharmaceutical composition of the above-mentioned (1);
(26) a commercial package comprising the composition of the above-mentioned (22) or (23) and written matter associated therewith, the written matter stating that the composition can or should be used for the prophylaxis or treatment of an HIV infectious disease or AIDS;
(27) the composition of the above-mentioned (15), wherein the piperidine compound having CCR5 antagonistic action is dissolved in at least one amphiphilic substance;
(28) the composition of the above-mentioned (15), wherein the piperidine compound having CCR5 antagonistic action is dispersed in at least one amphiphilic substance;
(29) the composition of the above-mentioned (15), wherein the amphiphilic substance comprises at least one kind of a dissolution aid;
(30) the composition of the above-mentioned (15), wherein the amphiphilic substance comprises at least one kind of a lipid;
(31) the composition of the above-mentioned (15), which is liquid at about 15°C to about 25°C;
(32) the composition of the above-mentioned (15), which is semi-solid at about 15°C to about 25°C;
(33) the composition of the above-mentioned (15), which is solid at about 15°C to about 25°C;
(34) the composition of the above-mentioned (15), which has a self-emulsifying action;
(35) the composition of the above-mentioned (15), wherein the amphiphilic substance comprises one or more kinds selected from water-soluble vitamin E derivatives, saturated polyglycolated glycerides, unsaturated polyglycolated glycerides, sorbitan fatty acid esters, polyglycerine fatty acid esters, polyoxyethylenepolypropylene glycols, polyoxyethylene castor oil derivatives and propylene glycol laurates in combination;
(36) the composition of the above-mentioned (15), wherein the amphiphilic substance comprises one or more kinds selected from water-soluble vitamin E derivatives, saturated polyglycolated glycerides and unsaturated polyglycolated glycerides;
(37) the composition of the above-mentioned (15), which comprises a water-soluble vitamin E derivative as an amphiphilic substance;
(38) the composition of the above-mentioned (37), wherein the vitamin E water-soluble derivative is D-α-tocopherol polyethylene glycol 1000 succinate;
(39) the composition of the above-mentioned (15), wherein the emulsion is formed by adding water to a system wherein a piperidine compound having a CCR5 antagonistic action is dissolved or dispersed in at least one amphiphilic substance;
(40) the composition of the above-mentioned (15), comprising the piperidine compound having CCR5 antagonistic action, a protease inhibitor and/or a reverse transcriptase inhibitor in combination;
(41) a capsule comprising the composition of the above-mentioned (15);
(42) the composition of the above-mentioned (15), which comprises water;
(43) the composition of the above-mentioned (15), which is a prophylactic or therapeutic agent of an HIV infectious disease;
(44) the composition of the above-mentioned (15), which is a prophylactic or therapeutic agent of AIDS;
(45) a method of prophylaxis or treatment of HIV infection, which comprises administration of the oral pharmaceutical composition of the above-mentioned (15) to a mammal;
(46) use of the composition of the above-mentioned (15) for the production of a prophylactic or therapeutic agent of an HIV infectious disease or AIDS;
(47) a commercial package comprising the composition of the above-mentioned (43) or (44) and written matter associated therewith, the written matter stating that the composition can or should be used for the prophylaxis or treatment of an HIV infectious disease or AIDS; and the like.

### Brief Description of the Drawings

Fig. 1 shows a comparison of the composition of the present invention and a conventional suspension in blood concentration of the compounds in Experimental Example 1.
Fig. 2 shows a comparison of the composition of the present invention and a conventional suspension in blood concentration of the compounds in Experimental Example 2.

### Detailed Description of the Invention

In the compound of the formula (I) to be used in the present invention, which has CCR5 antagonistic action, the hydrocarbon group represented by R¹ includes, for example, a chain aliphatic hydrocarbon group, an alicyclic hydrocarbon group, an aryl group and the like. Preferably, it is a chain aliphatic hydrocarbon group or an alicyclic hydrocarbon group.

The chain aliphatic hydrocarbon group includes, for example, a linear or branched aliphatic hydrocarbon group such as alkyl group, alkenyl group, alkynyl group and the like, with preference given to alkyl group. Examples of the alkyl group include C₁₋₁₀ alkyl groups, such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like (preferably C₁₋₆ alkyl etc.). Examples of the alkenyl group include C₂₋₆ alkenyl groups, such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Examples of the alkynyl group include C₂₋₆ alkynyl groups, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

Examples of the alicyclic hydrocarbon group include saturated or unsaturated alicyclic hydrocarbon groups, such as cycloalkyl group, cycloalkenyl group, cycloalkanedienyl group and the like, with preference given to cycloalkyl groups. Examples of the cycloalkyl group include C₃₋₉ cycloalkyl (preferably C₃₋₈ cycloalkyl etc.), such as cyclopropyl, cyclobutyl, cyclopentyl, cyclohexyl, cycloheptyl, cyclooctyl, cyclononyl and the like, and condensed rings such as 1-indanyl, 2-indanyl and the like. Examples of the cycloalkenyl group include C₃₋₆ cycloalkenyl groups, such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. Examples of the cycloalkanedienyl group include C₄₋₆ cycloalkanedienyl groups, such as 2,4-cyclopentanedien-1-yl, 2,4-cyclohexanedien-1-yl, 2,5-cyclohexanedien-1-yl and the like.

Examples of the aryl group include monocyclic or condensed polycyclic aromatic hydrocarbon groups, such as C₆₋₁₄ aryl groups, which are preferably phenyl, naphthyl, anthryl, phenanthryl, acenaphthylenyl, 4-indanyl, 5-indanyl etc., and the like, with particular preference given to phenyl, 1-naphthyl, 2-naphthyl and the like.

Examples of the "non-aromatic heterocyclic group" of the "nonaromatic heterocyclic group optionally having substituent(s)" represented by R¹ include 3- to 8-membered (preferably 5- or 6-membered) saturated or unsaturated (preferably saturated) non-aromatic heterocyclic groups (aliphatic heterocyclic groups), such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl etc., and the like.

Examples of the "substituent" of the hydrocarbon group optionally having substituent(s) as represented by R¹ and the substituent of the heterocyclic group optionally having substituent(s) as represented by R¹ include optionally substituted alkyl group, optionally substituted alkenyl group, optionally substituted alkynyl group, optionally substituted aryl group, optionally substituted cycloalkyl group or cycloalkenyl group, optionally substituted heterocyclic group, optionally substituted amino group, optionally substituted imidoyl group, optionally substituted amidino group, optionally substituted hydroxy group, optionally substituted thiol group, optionally esterified carboxyl group, optionally substituted carbamoyl group, optionally substituted thiocarbamoyl group, optionally substituted sulfamoyl group, halogen atom (e.g., fluorine, chlorine, bromine, iodine etc., preferably chlorine, bromine etc.), cyano group, nitro group, acyl group derived from sulfonic acid, acyl group derived from carboxylic acid, optionally substituted alkyl sulfinyl group, optionally substituted alkyl sulfonyl group, optionally substituted arylsulfinyl group, optionally substituted arylsulfonyl group and the like, wherein 1 to 5 (preferably 1 to 3) of these optional substituents may be present at substitutable position(s).

As the aryl group of the "optionally substituted aryl group" as a substituent, for example, C₆₋₁₄ aryl group such as phenyl, naphthyl, anthryl, phenanthryl, acenaphthylenyl and the like, and the like can be mentioned. Here, as the substituent of the aryl group, lower alkoxy group optionally substituted by halogen (e.g., C₁₋₆ alkoxy group such as methoxy, ethoxy, propoxy and the like, for example, halogen substituted C₁₋₄ alkoxy group such as fluoromethoxy, difluoromethoxy, trifluoromethoxy, 1,1-difluoroethoxy, 2,2-difluoroethoxy, 3,3-difluoropropoxy, 2,2,3,3,3-pentafluoropropoxy etc., and the like), aryloxy optionally having substituent(s) (e.g., phenoxy, 4-fluorophenoxy, 2-carbamoylphenoxy etc.), halogen atom (e.g., fluorine, chlorine, bromine, iodine etc.), lower alkyl group optionally having substituent(s) (e.g., unsubstituted C₁₋₆ alkyl group such as methyl, ethyl, propyl, and the like, for example, halogen-substituted C₁₋₄ alkyl group such as fluoromethyl, difluoromethyl, trifluoromethyl, 1,1-difluoroethyl, 2,2-difluoroethyl, 3,3-difluoropropyl, 2,2,3,3,3-pentafluoropropyl etc., and the like), C₃₋₈ cycloalkyl (e.g., cyclopropyl, cyclobutyl, cyclopentyl, cyclohexyl, cycloheptyl and the like), amino group, monosubstituted amino (e.g., carbamoylamino, methylsulfonylamino, methylamino, ethylamino, propylamino and the like), disubstituted amino (e.g., dimethylamino, diethylamino, N-methyl-N-methylsulfonylamino, di(methylsulfonyl)amino and the like), carbamoyl group optionally substituted by C₁₋₆ alkyl (e.g., butylcarbamoyl and the like), formyl, C₂₋₆ alkanoyl group (e.g., C₂₋₆ alkanoyl such as acetyl, propionyl, butyryl etc., and the like), C₆₋₁₄ aryl group (e.g., phenyl, naphthyl and the like), C₆₋₁₄ arylcarbonyl (e.g., benzoyl, naphthoyl and the like), C₇₋₁₃ aralkylcarbonyl (e.g., benzylcarbonyl, naphthylmethylcarbonyl and the like), hydroxy group, alkanoyloxy (e.g., C₂₋₅ alkanoyloxy such as acetyloxy, propionyloxy, butyryloxy and the like), C₇₋₁₃ aralkylcarbonyloxy (e.g., benzylcarbonyloxy and the like), nitro group, optionally substituted sulfamoyl group (besides unsubstituted sulfamoyl group, for example, N-methylsulfamoyl and the like), optionally substituted arylthio group (e.g., phenylthio, 4-methylphenylthio and the like), -N=N-phenyl, cyano group, oxo group, amidino group, optionally esterified carboxyl group (besides free carboxyl group, for example, methoxycarbonyl, ethoxycarbonyl, t-butoxycarbonyl and the like, C₁₋₄ alkoxycarbonyl etc.), C₁₋₆ alkylthio, C₁₋₆ alkylsulfinyl, C₁₋₆ alkylsulfonyl, C₆₋₁₄ arylthio, C₆₋₁₄ arylsulfinyl, C₆₋₁₄ arylsulfonyl, heterocyclic group optionally having substituent(s) (e.g., besides pyridyl, thienyl, tetrazolyl, morpholinyl, oxazolyl and the like, those mentioned below as the heterocyclic group for R³, which is optionally having substituent(s), and the like, wherein 1 or 2 of these optional substituents may be present at substitutable position(s).

The cycloalkyl group of the "optionally substituted cycloalkyl group" as a substituent may be, for example, C₃₋₇ cycloalkyl group, such as cyclopropyl, cyclobutyl, cyclopentyl, cyclohexyl, cycloheptyl etc., and the like. As used herein, examples of the substituent of the "cycloalkyl group" are similar in the kind and the number to those exemplified for the substituent of the aforementioned "optionally substituted aryl group".

The cycloalkenyl group of the "optionally substituted cycloalkenyl group" as a substituent may be, for example, C₃₋₆ cycloalkenyl group such as cyclopropenyl, cyclobutenyl, cyclopentenyl, cyclohexenyl etc., and the like. As used herein, examples of the substituent of the "optionally substituted cycloalkenyl group" are similar in the kind and the number to those exemplified for the substituent of the aforementioned "optionally substituted aryl group".

The alkyl group of the "optionally substituted alkyl group" as a substituent may be, for example, C₁₋₆ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl etc., and the like. As used herein, examples of the substituent of the alkyl group are similar in the kind and the number to those exemplified for the substituent of the aforementioned "optionally substituted aryl group".

The alkenyl group of the "optionally substituted alkenyl group" as a substituent may be, for example, C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc., and the like. As used herein, examples of the substituent of the alkenyl group are similar in the kind and the number to those exemplified for the substituent of the aforementioned "optionally substituted aryl group".

The alkynyl group of the "optionally substituted alkynyl group" as a substituent may be, for example, C₂₋₆ alkynyl group, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. As used herein, examples of the substituent of the alkynyl group are similar in the kind and the number to those exemplified for the substituent of the aforementioned "optionally substituted aryl group".

The heterocyclic group of the "optionally substituted heterocyclic group" as a substituent may be, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) hetero atoms which are of 1 to 3 kinds (preferably 1 or 2 kinds) selected from oxygen atom, sulfur atom and nitrogen atom, and the like.

Examples of the "aromatic heterocyclic group" include aromatic monocyclic heterocyclic group (e.g., 5- or 6-membered aromatic monocyclic heterocyclic group, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and condensed aromatic heterocyclic group [e.g., 8- to 12-membered condensed aromatic heterocycle (preferably a heterocycle wherein the aforementioned 5- or 6-membered aromatic monocyclic heterocyclic group is condensed with a benzene ring or a heterocycle wherein the same or different two heterocycles of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzoisothiazolyl, benzodioxolyl, benzimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinylt γ-carbolinyl, acridinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, pyrazolo[3,4-b]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

As the substituent that the "optionally substituted heterocyclic group" as a substituent may have, for example, lower alkyl group (e.g., C₁₋₆ alkyl group such as methyl, ethyl, propyl etc., and the like), acyl group (e.g., C₁₋₆ alkanoyl such as formyl, acetyl, propionyl, pivaloyl and the like, for example, C₇₋₁₃ arylcarbonyl such as benzoyl and the like, for example, C₁₋₆ alkylsulfonyl such as methylsulfonyl, ethylsulfonyl and the like, for example, substituted sulfonyl such as aminosulfonyl, methylaminosulfonyl, dimethylaminosulfonyl etc., and the like), halogenated lower alkyl (e.g., halogenated C₁₋₄ alkyl such as trifluoromethyl, 1,1-difluoroethyl and the like), and the like can be mentioned.

The substituent of the "optionally substituted amino group", "optionally substituted imidoyl group", "optionally substituted amidino group", "optionally substituted hydroxy group" and "optionally substituted thiol group" as a substituent may be, for example, lower alkyl group (e.g., C₁₋₆ alkyl group such as methyl, ethyl, propyl, isopropyl, butyl, isobutyl, t-butyl, pentyl, hexyl etc. etc.), aryl group (e.g., C₆₋₁₂ aryl group such as phenyl etc., C₁₋₄ alkyl group-substituted C₆₋₁₂ aryl such as 4-methylphenyl), C₃₋₈ cycloalkyl optionally having halogen or C₁₋₆ alkoxy as substituents, C₁₋₆ alkoxy group, acyl group (e.g., C₁₋₆ alkanoyl (e.g., formyl, acetyl, propionyl, pivaloyl etc.), C₇₋₁₃ arylcarbonyl such as benzoyl etc.), C₁₋₆ alkyl sulfonyl (e.g., methanesulfonyl, ethanesulfonyl etc.), C₆₋₁₄ arylsulfonyl such as p-toluenesulfonyl etc., optionally halogenated C₁₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl etc.), and the like. The "amino group" of the "optionally substituted amino group" as the substituent may be substituted by optionally substituted imidoyl group (e.g., C₁₋₆ alkylimidoyl, formylimidoyl, amidino etc.), and the like. In addition, two substituents may form a cyclic amino group together with a nitrogen atom. In this case, examples of the cyclic amino group include 3- to 8-membered (preferably 5-or 6-membered) cyclic amino, such as 1-azetidinyl, 1-pyrrolidinyl, 1-piperidinyl, 4-morpholinyl, 1-piperazinyl and 1-piperazinyl optionally having, at the 4-position, lower alkyl group (e.g., C₁₋₆ alkyl group, such as methyl, ethyl, propyl, isopropyl, butyl, t-butyl, pentyl, hexyl etc. etc.), aralkyl group (e.g., C₇₋₁₀aralkyl group, such as benzyl, phenethyl etc. etc.), aryl group (e.g., C₆₋₁₀ aryl group, such as phenyl, 1-naphthyl, 2-naphthyl etc. etc.), and the like.

Examples of the "optionally substituted carbamoyl group" include unsubstituted carbamoyl, N-monosubstituted carbamoyl group and N,N-disubstituted carbamoyl group.

The "N-monosubstituted carbamoyl group" is a carbamoyl group having one substituent on the nitrogen atom. Examples of the substituent include lower alkyl group (e.g., C₁₋₆ alkyl group, such as methyl, ethyl, propyl, isopropyl, butyl, isobutyl, t-butyl, pentyl, hexyl etc. etc.), cycloalkyl group (e.g., C₃₋₆ cycloalkyl group, such as cyclopropyl, cyclobutyl, cyclopentyl, cyclohexyl etc. etc.), aryl group (e.g., C₆₋₁₀ aryl group, such as phenyl, 1-naphthyl, 2-naphthyl etc. etc.), aralkyl group (e.g., C₇₋₁₀ aralkyl group, such as benzyl, phenethyl etc., preferably phenyl-C₁₋₄ alkyl group etc.), lower alkoxy group (e.g., C₁₋₆ alkoxy group such as methoxy, ethoxy, n-propoxy, isopropoxy, n-butoxy, isobutoxy, sec-butoxy, tert-butoxy and the like), heterocyclic group (e.g., those exemplified as the "heterocyclic group" as a substituent of "optionally substituted hydrocarbon group" for R¹ and the like). The lower alkyl group, cycloalkyl group, aryl group, aralkyl group, lower alkoxy and heterocyclic group may have substituents, which substituents are, for example, hydroxy group, optionally substituted amino group [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc. etc.), acyl group (e.g., C₁₋₆ alkanoyl such as formyl, acetyl, propionyl, pivaloyl etc., C₇₋₁₃ arylcarbonyl such as benzoyl, C₁₋₆ alkylsulfonyl such as methylsulfonyl, ethylsulfonyl etc.), and the like as substituents], halogen atom (e.g., fluorine, chlorine, bromine, iodine etc.), nitro group, cyano group, lower alkoxy group optionally substituted by 1 to 5 halogen atoms as substituents (e.g., fluorine, chlorine, bromine, iodine etc.) lower alkyl group optionally substituted by 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like, wherein the same or different 1 or 2 or 3 (preferably 1 or 2) substituents is(are) preferably used for the substitution.

The "N,N-disubstituted carbamoyl group" is a carbamoyl group having 2 substituents on a nitrogen atom. Examples of one of the substituents are those similar to the substituents of the aforementioned "N-monosubstituted carbamoyl group" and examples of the other include lower alkyl group (e.g., C₁₋₆ alkyl group, such as methyl, ethyl, propyl, isopropyl, butyl, t-butyl, pentyl, hexyl etc. etc.), C₃₋₆ cycloalkyl group (e.g., cyclopropyl, cyclobutyl, cyclopentyl, cyclohexyl etc.), C₇₋₁₀ aralkyl group (e.g., benzyl, phenethyl etc., preferably phenyl-C₁₋₄ alkyl group-etc.) and the like. Two substituents may form a cyclic amino group together with a nitrogen atom. In this case, examples of the cyclic aminocarbonyl group include 3- to 8-membered (preferably 5- or 6-membered) cyclic amino such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl optionally having, at the 4-position, lower alkyl group (e.g., C₁₋₆ alkyl group, such as methyl, ethyl, propyl, isopropyl, butyl, t-butyl, pentyl, hexyl etc. etc.), aralkyl group (e.g., C₇₋₁₀ aralkyl group, such as benzyl, phenethyl etc. etc.), aryl group (e.g., C₆₋₁₀ aryl group, such as phenyl, 1-naphthyl, 2-naphthyl etc. etc.), and the like.

Examples of the substituent of the "optionally substituted thiocarbamoyl group" are similar to those exemplified for the substituent of the aforementioned "optionally substituted carbamoyl group".

Examples of the "sulfamoyl group optionally having substituent(s)" include unsubstituted sulfamoyl, N-monosubstituted sulfamoyl group and N,N-disubstituted sulfamoyl group.

The "N-monosubstituted sulfamoyl group" means sulfamoyl group having one substituent on a nitrogen atom. Examples of the substituent are those similar to the substituent of the "N-monosubstituted carbamoyl group".

The "N,N-disubstituted sulfamoyl group" means sulfamoyl group having 2 substituents on a nitrogen atom. Examples of the substituent are those similar to the substituent of the "N,N-disubstituted carbamoyl group".

Examples of the "optionally esterified carboxyl group" include, besides free carboxyl group, lower alkoxy carbonyl group, aryloxycarbonyl group, aralkyloxycarbonyl group and the like.

Examples of the "lower alkoxy carbonyl group" include C₁₋ ₆ alkoxy-carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl etc., and the like. Of these, C₁₋₃ alkoxycarbonyl groups, such as methoxycarbonyl, ethoxycarbonyl, propoxycarbonyl etc., and the like are preferable.

Examples of the "aryloxycarbonyl group" preferably include C₇₋₁₂ aryloxy-carbonyl group, such as phenoxycarbonyl, 1-naphthoxycarbonyl, 2-naphthoxycarbonyl etc., and the like.

Examples of the "aralkyloxycarbonyl group" preferably include C₇₋₁₀ aralkyloxy-carbonyl groups such as benzyloxycarbonyl, phenethyloxycarbonyl etc., and the like (preferably C₆₋₁₀ aryl-C₁₋₄ alkoxy-carbonyl etc.).

The "lower alkoxycarbonyl", "aryloxycarbonyl group" and "aralkyloxycarbonyl group" may have substituents. Examples of the substituents are similar in the kind and the number to those exemplified for the substituent of aryl group and aralkyl group as the substituents of the aforementioned N-monosubstituted carbamoyl group.

As the "acyl group derived from sulfonic acid" as a substituent, hydrocarbon group and sulfonyl bonded to each other and the like can be mentioned. Preferably, it is acyl such as C₁₋₁₀ alkylsulfonyl, C₂₋₆ alkenylsulfonyl, C₂₋₆ alkynylsulfonyl, C₃₋₉ cycloalkylsulfonyl, C₃₋₉ cycloalkenylsulfonyl, C₆₋₁₄ arylsulfonyl, C₇₋₁₀ aralkylsulfonyl and the like. Specific C₁₋₁₀ alkyl here is, for example, methyl, ethyl, propyl, isopropyl, butyl, isobutyl, t-butyl, pentyl, hexyl, heptyl, octyl, and the like. As the C₂₋₆ alkenyl, for example, vinyl, allyl, 1-propenyl, isopropenyl, 2-butenyl, 3-butenyl, 2-hexenyl, and the like can be mentioned. As the C₂₋₆ alkynyl, for example, ethynyl, 2-propynyl, 2-butynyl, 5-hexynyl, and the like can be mentioned. As the C₃₋₉ cycloalkyl, for example, cyclopropyl, cyclobutyl, cyclopentyl, cyclohexyl, cyclooctyl, and the like can be mentioned. As the C₃₋₉ cycloalkenyl, for example, 1-cyclopenten-1-yl, 2-cyclopenten-1-yl, 3-cyclopenten-1-yl, 3-cyclohexen-1-yl, 3-cycloocten-1-yl, and the like can be mentioned. As the C₆₋₁₄ aryl, phenyl, 1-naphthyl, 2-naphthyl, and the like can be mentioned. As the C₇₋₁₀ aralkylsulfonyl, for example, benzyl, phenethyl, and the like can be mentioned. These hydrocarbon groups bonded with sulfonyl may have substituents, and as the substituent, for example, hydroxy group, optionally substituted amino group [wherein the amino group may have, as substituent(s), 1 or 2 of, for example, lower alkyl group (e.g., C₁₋₆ alkyl group such as methyl, ethyl, propyl, isopropyl, butyl, isobutyl, t-butyl, pentyl, hexyl etc. etc.), acyl group (e.g., C₁₋₆ alkanoyl such as formyl, acetyl, propionyl, pivaloyl etc., C₇₋₁₃ arylcarbonyl such as benzoyl etc., C₁₋₆ alkylsulfonyl such as methylsulfonyl, ethylsulfonyl etc.) and the like], halogen atom (e.g., fluorine, chlorine, bromine, iodine etc.), nitro group, cyano group, lower alkyl group optionally substituted by 1 to 5 halogen atoms (e.g., fluorine, chlorine, bromine, iodine etc.), lower alkoxy group optionally substituted by 1 to 5 halogen atoms (e.g., fluorine, chlorine, bromine, iodine etc.), and the like can be mentioned. As the lower alkyl group, for example, C₁₋₆ alkyl group such as methyl, ethyl, n-propyl, isopropyl, n-butyl, isobutyl, sec-butyl, tert-butyl, pentyl, hexyl and the like, and the like can be mentioned, with particular preference given to methyl, ethyl, and the like. As the lower alkoxy group, for example, C₁₋₆ alkoxy group such as methoxy, ethoxy, n-propoxy, isopropoxy, n-butoxy, isobutoxy, sec-butoxy, tert-butoxy and the like, and the like can be mentioned, with particular preference given to methoxy, ethoxy, and the like, wherein the same or different 1 or 2 or 3 (preferably 1 or 2) of these substituents is(are) preferably used for the substitution.

The "acyl group derived from carboxylic acid" as the substituent is exemplified by one wherein a hydrogen atom or the single substituent that the aforementioned "N-monosubstituted carbamoyl group" has on a nitrogen atom is bonded to carbonyl, and the like. Preferred are acyl, such as benzoyl, C₁₋₆ alkanoyl, e.g., formyl, acetyl, propionyl, pivaloyl etc., and C₇₋₁₃ arylcarbonyl such as benzoyl etc., and the like.

The alkyl of the "alkyl sulfinyl group optionally having substituent(s)" and "alkyl sulfonyl group optionally having substituent(s)" as the substituent may be, for example, lower alkyl groups such as C₁₋₆ alkyl group (e.g., methyl, ethyl, propyl, isopropyl, butyl, isobutyl, t-butyl, pentyl, hexyl etc.), and the like.

The aryl of the "arylsulfinyl group optionally having substituent(s)" as the substituent may be, for example, a C₆₋₁₄ aryl group, such as phenyl, naphthyl, anthryl, phenanthryl, acenaphthylenyl etc., and the like.

The substituent of these alkyl and aryl may be, for example, lower alkoxy group (e.g., C₁₋₆ alkoxy group, such as methoxy, ethoxy, propoxy etc. etc.), halogen atom (e.g., fluorine, chlorine, bromine, iodine etc.), lower alkyl group (e.g., C₁₋₆ alkyl group, such as methyl, ethyl, propyl etc. etc.), amino group, hydroxy group, cyano group, amidino group and the like, wherein one or two of these optional substituents may be present at a substitutable position.

As the "hydrocarbon group optionally having substituent(s)" and "nonaromatic heterocyclic group optionally having substituent(s)" represented by R², those similar to the "hydrocarbon group optionally having substituent(s)" and "nonaromatic heterocyclic group optionally having substituent(s)" represented by R¹ can be mentioned. Of those mentioned for R¹, preferred are C₂₋₆ alkyl optionally having substituent(s) and C₃₋₈ cycloalkyl optionally having substituent(s).

When R¹ and R² in combination form, together with an adjacent nitrogen atom, a ring optionally having substituent(s), the ring may contain, besides one nitrogen atom, a different nitrogen atom, oxygen atom, sulfur atom. Examples thereof include monocyclic groups, such as 1-azetidinyl, 1-pyrrolidinyl, 1-piperidinyl, 1-homopiperidinyl, heptamethyleneimino, 1-piperazinyl, 1-homopiperazinyl, 4-morpholinyl, 4-thiomorpholinyl and the like, condensed rings such as 2-isoindolinyl, 1,2,3,4-tetrahydro-2-isoquinolyl, 1,2,4,5-tetrahydro-3H-3-benzodiazepin-3-yl and the like, cyclic amino groups such as spiro ring and the like (e.g., indene-1-spiro-4'-piperidin-1'-yl etc.), said cyclic amino group optionally having 1 to 5, preferably 1 to 3, substituent(s) at a chemically permitted position on the ring.

Examples of the substituent include hydroxy group, cyano group, nitro group, oxo group, halogen atom, a group of the general formula -B¹R^{a} (wherein R^{a} is a hydrocarbon group optionally having substituent(s) or a heterocyclic group optionally having substituent(s), B¹ is a bond (single bond),-CR^{b}R^{c}-, -COO-, -CO-, -CR^{b}(OH)-, -CO-NR^{b}-, -CS-NR^{b}-, -CO-S-, -CS-S-, -CO-NR^{b}-CO-NR^{c}-, -C(=NH)-NR^{b}-, -NR^{b}-, -NR^{b}-CO-, -NR^{b}-CS-,-NR^{b}-CO-NR^{c}-, -NR^{b}-CS-NR^{c}-, -NR^{b}-CO-O-, -NR^{b}-CS-O-, -NR^{b}-CO-S-,-NR^{b}-CS-S-, -NR^{b}-C(=NH)-NR^{c}-, -NR^{b}-SO₂-, -NR^{b}-NR^{c}-, -O-, -O-CO-,-O-CS-, -O-CO-O, -O-CO-NR^{b}-, -O-C(=NH)-NR^{b}-, -S-, -SO-, -SO₂-,-CR^{b}R^{c}-S-, CR^{b}R^{c}-SO₂-, -SO₂-NR^{b}-, -S-CO-, -S-CS-, -S-CO-NR^{b}-, -S-CS-NR^{b}-, -S-C(=NH)-NR^{b}- and the like, wherein R^{b} and R^{c} are each a hydrogen atom, optionally substituted alkyl group, optionally substituted alkenyl group, optionally substituted alkynyl group, optionally substituted aryl group, optionally substituted cycloalkyl group or cycloalkenyl group, optionally substituted heterocyclic group, acyl group derived from sulfonic acid, acyl group derived from carboxylic acid etc.), and the like.

As the "hydrocarbon group" of the "hydrocarbon group optionally having substituent(s)" represented by the aforementioned R^{a}, for example, aliphatic chain hydrocarbon group, alicyclic hydrocarbon group and aryl group, and the like can be mentioned. As these aliphatic chain hydrocarbon group, alicyclic hydrocarbon group and aryl group, those mentioned above as the aliphatic chain hydrocarbon group, alicyclic hydrocarbon group and aryl group represented by R¹ can be respectively mentioned. As the substituent of the hydrocarbon group, those mentioned above as the substituent of the "hydrocarbon group optionally having substituent(s)" represented by R¹ can be mentioned.

As the "heterocyclic group" of the "heterocyclic group optionally having substituent(s)" represented by the above-mentioned R^{a}, those similar to the "heterocyclic group" of the "heterocyclic group optionally having substituent(s)" to be shown below for R³ can be mentioned. As the "substituent" of the "optionally substituted heterocyclic group", those mentioned above as the "substituent" of the "nonaromatic hydrocarbon group optionally having substituent(s)" represented by R¹ can be mentioned.

As the optionally substituted alkyl group, optionally substituted alkenyl group, optionally substituted alkynyl group, optionally substituted aryl group, optionally substituted cycloalkyl group or cycloalkenyl group, optionally substituted heterocyclic group, acyl group derived from sulfonic acid and acyl group derived from carboxylic acid, as represented by the aforementioned R^{b} and R^{c}, those exemplified as the substituent of the hydrocarbon group optionally having substituent(s) for the above-mentioned R¹ are exemplified.

It is preferable that R¹ and R² in combination form, together with an adjacent nitrogen atom, a heterocycle optionally having substituent(s).

More preferably, NR¹R² is a group of the formula 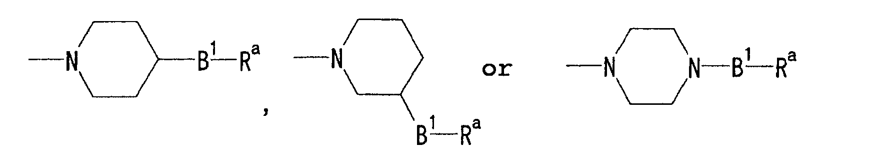 wherein B¹ and R^{a} are as defined above. As used here, B¹ and R^{a} are as defined above, and R^{a} is particularly preferably an aryl group optionally having substituent(s) or a heterocyclic group optionally having substituent(s).

As the "cyclic hydrocarbon group" of the "cyclic hydrocarbon group optionally having substituent(s)" represented by R³, alicyclic hydrocarbon group, aryl group and the like can be mentioned.

As the alicyclic hydrocarbon group, for example, saturated or unsaturated alicyclic hydrocarbon group such as cycloalkyl group, cycloalkenyl group, cycloalkanedienyl group and the like can be mentioned. Preferred is cycloalkyl group. As the cycloalkyl group, for example, C₃₋₉ cycloalkyl such as cyclopropyl, cyclobutyl, cyclopentyl, cyclohexyl, cycloheptyl, cyclooctyl, cyclononyl and the like (preferably C₃₋₈ cycloalkyl etc.), and the like, and a fused ring such as 1-indanyl, 2-indanyl and the like can be mentioned. As the cycloalkenyl group, for example, C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl etc., and the like can be mentioned. As the cycloalkanedienyl group, for example, C₄₋₆ cycloalkanedienyl group such as 2,4-cyclopentanedien-1-yl, 2,4-cyclohexanedien-1-yl, 2,5-cyclohexanedien-1-yl etc., arid the like can be mentioned.

As the aryl group, a monocyclic or condensed polycyclic aromatic hydrocarbon group can be mentioned. For example, C₆₋₁₄ aryl group such as phenyl, naphthyl, anthryl, phenanthryl, acenaphtylenyl, 4-indanyl, 5-indanyl etc., and the like are preferable, and phenyl, 1-naphthyl, 2-naphthyl, and the like are particularly preferable.

As the substituent of the cyclic hydrocarbon group optionally having substituent(s) represented by R³, those mentioned above as the substituent of the hydrocarbon group optionally having substituent(s) represented by R¹ can be mentioned. When the cyclic hydrocarbon group is an alicyclic hydrocarbon group, for example, phenyl group, phenyl group optionally substituted by C₁₋₆ alkyl such as tolyl group etc., naphthyl group and the like can be mentioned. When the cyclic hydrocarbon group is an aryl group, for example, halogen atom (e.g., chlorine atom, fluorine atom etc.), C₁₋₆ alkyl group (methyl, ethyl, propyl, isopropyl, butyl, isobutyl, t-butyl, pentyl, hexyl etc.), C₁₋₆ alkoxy group (e.g., methoxy, ethoxy, n-propoxy, isopropoxy, n-butoxy etc.), C₃₋₆ cycloalkyl group (e.g., cyclopropyl, cyclobutyl, cyclopentyl, cyclohexyl etc.), C₁₋₆ alkyl halide group (trifluoromethyl etc.), C₁₋₆ alkoxy halide group (trifluoromethyloxy etc.), C₁₋₆ alkylthio group (methylthio, ethylthio etc.), C₁₋₆ alkylsulfonyl group (methylsulfonyl, ethylsulfonyl etc.), cyano group, nitro group and the like can be mentioned.

The heterocyclic group of the heterocyclic group optionally having substituent(s) for R³ is, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom, a nitrogen atom and the like.

Examples of the aromatic heterocyclic group include aromatic monocyclic heterocyclic group (e.g., 5- or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.); condensed aromatic heterocyclic group [e.g., 8- to 12-membered condensed aromatic heterocyclic group (preferably a heterocycle wherein the aforementioned 5-or 6-membered aromatic monocyclic heterocyclic group is condensed with a benzene ring or a heterocycle wherein the same or different two heterocycles of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolihyl, β-carbolinyl, γ-carbolinyl, acridinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the non-aromatic heterocyclic group include 3- to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl etc., and the like.

As the substituent of the "heterocyclic group optionally having substituent(s)" represented by R³, those mentioned above as the "substituent" of the "nonaromatic heterocyclic group optionally having substituent(s)" represented by R¹ can be mentioned.

As the R³, phenyl group optionally having substituent(s) is preferable.

As the hydrocarbon group optionally having substituent(s) represented by R⁴, those mentioned above as the hydrocarbon group optionally having substituent(s) represented by R¹ can be mentioned, and as the heterocyclic group optionally having substituent(s) represented by R⁴, those similar to the heterocyclic group optionally having substituent(s) represented by R³ can be mentioned.

As the "alkoxy group" of the "alkoxy group optionally having substituent(s)" represented by R⁴, for example, C₁₋₆ alkoxy such as methoxy, ethoxy, n-propoxy, isopropoxy, n-butoxy, isobutoxy, sec-butoxy, tert-butoxy and the like is preferable, and as the substituent, for example, cycloalkyl group (e.g., C₃₋₆ cycloalkyl group, such as cyclopropyl, cyclobutyl, cyclopentyl, cyclohexyl etc. etc.), aryl group (e.g., C₆₋₁₀ aryl group, such as phenyl, 1-naphthyl, 2-naphthyl etc. etc.), aralkyl group (e.g., C₇₋₁₀ aralkyl group, such as benzyl, phenethyl etc., preferably phenyl-C₁₋₄ alkyl group etc.), heterocyclic group (e.g., those exemplified as the "heterocyclic group" as a substituent of "optionally substituted hydrocarbon group" for R¹ and the like). The cycloalkyl group, aryl group, aralkyl group and heterocyclic group may have substituents, which substituents are, for example, hydroxy group, optionally substituted amino group [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc. etc.), acyl group (e.g., C₁₋₆ alkanoyl such as formyl, acetyl, propionyl, pivaloyl etc., C₇₋₁₃ alkylcarbonyl such as benzoyl etc.), C₁₋₆ alkylsulfonyl such as methylsulfonyl, ethylsulfonyl etc. and the like as substituents], halogen atom (e.g., fluorine, chlorine, bromine, iodine etc.), nitro group, cyano group, lower alkoxy group optionally substituted by 1 to 5 halogen atoms as substituents (e.g., fluorine, chlorine, bromine, iodine etc.) lower alkyl group optionally substituted by 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like, wherein the same or different 1 or 2 or 3 (preferably 1 or 2) of these substituents is(are) preferably used for the substitution.

As the "aryl group" of the "optionally substituted aryloxy group" represented by R⁴, for example, C₆₋₁₄ aryl group such as phenyl, naphthyl, anthryl, phenanthryl, acenaphthylenyl and the like, and the like can be mentioned, and as the "substituent", lower alkoxy group (e.g., C₁₋₆ alkoxy group such as methoxy, ethoxy, propoxy etc., and the like), halogen atom (e.g., fluorine, chlorine, bromine, iodine etc.), lower alkyl group (e.g., C₁₋₆ alkyl group such as methyl, ethyl, propyl etc., and the like), amino group, hydroxy group, cyano group, amidino group, and the like can be mentioned, wherein these optional substituents may have 1 or 2 substituents at substitutable position(s).

As the "amino group optionally having substituent(s)" represented by R⁴, for example, lower alkyl group (e.g., C₁₋₆ alkyl group, such as methyl, ethyl, propyl, isopropyl, butyl, isobutyl, t-butyl, pentyl, hexyl etc. etc.), acyl group (e.g., C₁₋₆ alkanoyl (e.g., formyl, acetyl, propionyl, pivaloyl etc.), benzoyl etc.), C₁₋₆ alkylsulfonyl (e.g., methanesulfonyl, ethanesulfonyl etc.), optionally halogenated C₁₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl etc.), and the like can be mentioned. The "amino group" of the "amino group optionally having substituent(s)" as the substituent may be substituted by imidoyl group optionally having substituent(s) (e.g., C₁₋₆ alkyl imidoyl, formylimidoyl, amidino etc.), and the like. In addition, two substituents may form a cyclic amino group together with a nitrogen atom. In this case, examples of the cyclic amino group include 3- to 8-membered (preferably 5- or 6-membered) cyclic amino, such as 1-azetidinyl, 1-pyrrolidinyl, 1-piperidinyl, 4-morpholinyl, 1-piperazinyl and 1-piperazinyl optionally having, at the 4-position, lower alkyl group (e.g., C₁₋₆ alkyl group, such as methyl, ethyl, propyl, isopropyl, butyl, t-butyl, pentyl, hexyl etc. etc.), aralkyl group (e.g., C₇₋₁₀ aralkyl group, such as benzyl, phenethyl etc. etc.), aryl group (e.g., C₆₋₁₀ aryl group, such as phenyl, 1-naphthyl, 2-naphthyl etc. etc.), and the like.

As R⁴, C₁₋₃ alkyl, phenyl optionally having substituent(s), 3-pyridyl, 4-pyridyl, and the like are preferable.

As the hydrocarbon group represented by R⁵, those mentioned as the "hydrocarbon group" of the "hydrocarbon group optionally having substituent(s)" represented by R¹ can be mentioned. Preferred are, for example, lower (C₁₋₄) alkyl such as methyl, ethyl, n-propyl, isopropyl, butyl, n-butyl, isobutyl, tert-butyl and the like.

As the counter anion represented by Y⁻, for example, Cl⁻, Br⁻, I⁻, NO₃⁻, SO₄²⁻, PO₄³⁻, CH₃SO₃⁻ and the like can be mentioned.

The divalent chain hydrocarbon group of the divalent chain hydrocarbon group optionally having substituent(s) other than oxo group, as represented by E, is exemplified by C₁₋₆ alkylene, such as methylene, ethylene etc., C₂₋₆ alkenylene, such as ethenylene etc., C₂₋₆ alkynylene, such as ethynylene etc., and the like. Preferred is C₂₋₅ alkylene and more preferred is trimethylene.

The substituent of the divalent hydrocarbon group may be any as long as it is not an oxo group. Examples thereof include optionally substituted alkyl group, optionally substituted aryl group, optionally substituted cycloalkyl group or cycloalkenyl group, optionally esterified carboxyl group, optionally substituted carbamoyl group or thiocarbamoyl group, optionally substituted amino group, optionally substituted hydroxy group, optionally substituted thiol (mercapto) group, acyl group derived from carboxylic acid, acyl group derived from sulfonic acid, halogen (e.g., fluorine, chlorine, bromine etc.), nitro, cyano and the like. The number of the substituents may be 1 to 3. The optionally substituted alkyl group, an optionally substituted aryl group, optionally substituted cycloalkyl group or cycloalkenyl group, optionally esterified carboxyl group, optionally substituted carbamoyl group or thiocarbamoyl group, optionally substituted amino group, optionally substituted hydroxy group, optionally substituted thiol (mercapto) group, acyl group derived from carboxylic acid, acyl group derived from sulfonic acid are those similar to the substituent of the heterocyclic group optionally having substituent(s) as represented by the aforementioned R³.

The divalent chain C₁₋₃ hydrocarbon group of the divalent chain C₁₋₃ hydrocarbon group optionally having substituent(s), as represented by Q and R, is exemplified by one having 1 to 3 carbon atoms from the divalent chain hydrocarbon group of the divalent chain hydrocarbon group optionally having substituent(s) other than oxo group, as represented by E.

The substituent of the divalent chain C₁₋₃ hydrocarbon group optionally having substituent(s), as represented by Q and R, is exemplified by those exemplified as the substituent of the divalent chain hydrocarbon group optionally having substituent(s) other than oxo group, as represented by E.

J is methine or a nitrogen atom, with preference given to methine.

G¹ is a bond, CO or SO₂, with preference given to CO or SO₂.

G² is CO, SO₂, NHCO, CONH or OCO, with preference given to CO, NHCO and OCO.

As the "hydrocarbon group" of the "hydrocarbon group optionally having substituent(s)" represented by R₁, for example, aliphatic chain hydrocarbon group, alicyclic hydrocarbon group and aryl group, and the like can be mentioned. As the aliphatic chain hydrocarbon group, for example, C₁₋₆ alkyl group such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl and the like, and the like are preferable; as the alicyclic hydrocarbon group, for example, C₃₋₈ cycloalkyl group such as cyclopropyl, cyclobutyl, cyclopentyl, cyclohexyl, cycloheptyl, cyclooctyl and the like, and the like are preferable; and as the aryl group, for example, C₆₋₁₄ aryl group such as phenyl, naphthyl (1-naphthyl, 2-naphthyl) and the like, and the like are preferable.

As the "substituent" of the "hydrocarbon group optionally having substituent(s)" represented by R₁, hydrocarbon group optionally having substituent(s), heterocyclic group optionally having substituent(s), halogen atom (e.g., fluorine, chlorine, bromine, iodine), C₁₋₄ alkoxy group optionally having substituent(s), C₁₋₄ alkylthio group optionally having substituent(s), C₂₋₆ alkoxycarbonyl group optionally having substituent(s), C₁₋₆ alkanoyl group optionally having substituent(s), amino group optionally having substituent(s), nitro group, cyano group, carbamoyl group optionally having substituent(s), sulfamoyl group optionally having substituent(s), acyl group derived from sulfonic acid and the like can be mentioned.

As the "hydrocarbon group" of the "hydrocarbon group optionally having substituent(s)", those similar to the "hydrocarbon group" of the "hydrocarbon group optionally having substituent(s)" represented by R₁ can be mentioned, of which C₁₋₆ alkyl group, C₃₋₈ cycloalkyl group and C₆₋₁₄ aryl group are preferable. These are also exemplified by those mentioned for R¹. As the "substituent" of the "hydrocarbon group optionally having substituent(s)", for example, lower alkoxy group (e.g., C₁₋₆ alkoxy group such as methoxy, ethoxy, propoxy etc., and the like), halogen atom (e.g., fluorine, chlorine, bromine, iodine etc.), lower alkyl group (e.g., C₁₋₆ alkyl group such as methyl, ethyl, propyl etc., and the like), lower alkynyl group (e.g., C₁₋₄ alkynyl group such as vinyl, 1-propenyl, 2-propenyl, isopropenyl, butenyl, isobutenyl and the like), amino group, hydroxy group, cyano group, amidino group, and the like can be mentioned, wherein 1 or 3 of these optional substituents may be present at substitutable position(s).

The heterocyclic group of the "heterocyclic group optionally having substituent(s)" (substituent of the hydrocarbon group optionally having substituent(s) represented by R₁) may be, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom and a nitrogen atom, and the like. Examples of the "aromatic heterocyclic group" include aromatic monocyclic heterocyclic group (e.g., 5- or 6-membered aromatic monocyclic heterocyclic group, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and condensed aromatic heterocyclic group [e.g., 8- to 12-membered condensed aromatic heterocycle (preferably a heterocycle wherein the aforementioned 5- or 6-membered aromatic monocyclic heterocyclic group is condensed with a benzene ring or a heterocycle wherein the same or different two heterocycle of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, benzodioxolyl, benzimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxa-zinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-a]pyridyl, imidazo[1,2-b]pyridazinyl, imidazo [1,2-a]pyrimidinyl, 1,2,4-triazolo[4,3-a]pyridyl, 1,2,4-triazolo[4,3-b]pyridazinyl etc.] and the like.

Examples of the "non-aromatic heterocyclic group" include 3- to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl etc., and the like.

As the "substituent" of the "optionally substituted heterocyclic group" (substituent of the hydrocarbon group optionally having substituent(s) represented by R₁) may be, for example, those similar to the "substituent" of the hydrocarbon group optionally having substituent(s)", which are the "substituent" of the hydrocarbon group optionally having substituent(s) represented by R₁a can be mentioned.

As the "C₁₋₄ alkoxy group" of the "C₁₋₄ alkoxy group optionally having substituent(s)", for example, methoxy, ethoxy, n-propoxy, isopropoxy, n-butoxy, isobutoxy, tert-butoxy and the like can be mentioned; as the "C₁₋₄ alkylthio group" of the "C₁₋₄ alkylthio group optionally having substituent(s)", for example, methylthio, ethylthio, n-propylthio, isopropylthio, n-butylthio, isobutylthio, tert-butylthio and the like can be mentioned; and as the "C₂₋₆ alkoxycarbonyl group" of the "C₂₋₆ alkoxycarbonyl group optionally having substituent(s)", for example, methoxycarbonyl, ethoxycarbonyl, n-propoxycarbonyl, isopropoxycarbonyl, n-butoxycarbonyl, isobutoxycarbonyl, tert-butoxycarbonyl, n-pentyloxycarbonyl and the like can be mentioned.

As the "C₁₋₆ alkanoyl group" of the "C₁₋₆ alkanoyl group optionally having substituent(s)", for example, formyl, acetyl, propionyl, pivaloyl and the like can be mentioned. As the substituent of the "C₁₋₄ alkoxy group optionally having substituent(s)", "C₁₋₄ alkylthio group optionally having substituent(s)", "C₁₋₆ alkoxycarbonyl group optionally having substituent(s)", and "C₁₋₆ alkanoyl group optionally having substituent(s)", those similar to the substituent of the "hydrocarbon group optionally having substituent(s)", which are substituents of the hydrocarbon group optionally having substituent(s) represented by R₁ can be mentioned.

As the substituent of the "amino group optionally having substituent(s)", for example, lower alkyl group (e.g., C₁₋₆ alkyl group such as methyl, ethyl, propyl, isopropyl, butyl, isobutyl, t-butyl, pentyl, hexyl etc. etc.), acyl group derived from carboxylic acid (e.g., C₁₋₆ alkanoyl such as formyl, acetyl, propionyl, pivaloyl and the like), C₇₋₁₅ arylcarbonyl such as benzoyl etc. etc.), acyl group derived from sulfonic acid (e.g., C₁₋₆ alkylsulfonyl such as methylsulfonyl, ethylsulfonyl etc.), optionally halogenated C₂₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl etc.), and the like can be mentioned. The "amino group" of the "amino group optionally having substituent(s)" may be substituted by optionally substituted imidoyl group (e.g., C₁₋₆ alkylimidoyl, formylimidoyl, amidino etc.) and the like and 2 substituents may form a cyclic amino group together with nitrogen atom. As the cyclic amino group in such cases, for example, a 3- to 8-membered (preferably 5-or 6-membered) cyclic amino group such as 1-azetidinyl, 1-pyrrolidinyl, 1-piperidinyl, 4-morpholinyl, 1-piperazinyl and 1-piperazinyl optionally having, at the 4-position, lower alkyl group (e.g., C₁₋₆ alkyl group such as methyl, ethyl, propyl, isopropyl, butyl, t-butyl, pentyl, hexyl etc., and the like), aralkyl group (e.g., C₇₋₁₀ aralkyl group such as benzyl, phenethyl etc. and the like), aryl group (e.g., C₆₋₁₀ aryl group such as phenyl, 1-naphthyl, 2-naphthyl etc., and the like), and the like, and the like can be mentioned.

Examples of the "carbamoyl group optionally having substituent(s)" include unsubstituted carbamoyl, N-monosubstituted carbamoyl group and N,N-disubstituted carbamoyl group.

The "N-monosubstituted carbamoyl group" is a carbamoyl group having one substituent on the nitrogen atom. Examples of the substituent include lower alkyl group (e.g., C₁₋₆ alkyl group, such as methyl, ethyl, propyl, isopropyl, butyl, isobutyl, t-butyl, pentyl, hexyl etc. etc.), cycloalkyl group (e.g., C₃₋₆ cycloalkyl group, such as cyclopropyl, cyclobutyl, cyclopentyl, cyclohexyl etc. etc.), aryl group (e.g., C₆₋₁₀ aryl group, such as phenyl, 1-naphthyl, 2-naphthyl etc. etc.), aralkyl group (e.g., C₇₋₁₀ aralkyl group, such as benzyl, phenethyl etc., preferably phenyl-C₁₋₄ alkyl group etc.), lower alkoxy group (e.g., C₁₋₆ alkoxy group such as methoxy, ethoxy, n-propoxy, isopropoxy, n-butoxy, isobutoxy, sec-butoxy, tert-butoxy and the like), heterocyclic group (e.g., those exemplified as the "heterocyclic group" of the "optionally substituted heterocyclic group" as a substituent of "optionally substituted hydrocarbon group" for R¹ and the like). The lower alkyl group, lower alkoxy group, cycloalkyl group, aryl group, aralkyl group, lower alkoxy and heterocyclic group may have substituents, which substituents are, for example, hydroxy group, optionally substituted amino group [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc. etc.), acyl group (e.g., C₁₋₆ alkanoyl such as formyl, acetyl, propionyl, pivaloyl etc., C₇₋₁₃ arylcarbonyl such as benzoyl, C₁₋₆ alkylsulfonyl such as methylsulfonyl, ethylsulfonyl etc.), and the like as substituents], halogen atom (e.g., fluorine, chlorine, bromine, iodine etc.), nitro group, cyano group, lower alkoxy group optionally substituted by 1 to 5 halogen atoms as substituents (e.g., fluorine, chlorine, bromine, iodine etc.) lower alkyl group optionally substituted by 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like, wherein the same or different 1 or 2 or 3 (preferably 1 or 2) of these substituents is(are) preferably used for the substitution.

The "N,N-disubstituted carbamoyl group" is a carbamoyl group having 2 substituents on a nitrogen atom. Examples of one of the substituents are those similar to the substituents of the aforementioned "N-monosubstituted carbamoyl group" and examples of the other include lower alkyl group (e.g., C₁₋₆ alkyl group, such as methyl, ethyl, propyl, isopropyl, butyl, t-butyl, pentyl, hexyl etc. etc.), C₃₋₆ cycloalkyl group (e.g., cyclopropyl, cyclobutyl, cyclopentyl, cyclohexyl etc.), C₇₋₁₀ aralkyl group (e.g., benzyl, phenethyl etc., preferably phenyl-C₁₋₄ alkyl group etc.) and the like. Two substituents may form a cyclic amino group together with a nitrogen atom. In this case, examples of the cyclic aminocarbonyl group include 3- to 8-membered (preferably 5- or 6-membered) cyclic amino such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl optionally having, at the 4-position, lower alkyl group (e.g., C₁₋₆ alkyl group, such as methyl, ethyl, propyl, isopropyl, butyl, t-butyl, pentyl, hexyl etc. etc.), aralkyl group (e.g., C₇₋₁₀ aralkyl group, such as benzyl, phenethyl etc. etc.), aryl group (e.g., C₆₋₁₀ aryl group, such as phenyl, 1-naphthyl, 2-naphthyl etc. etc.), and the like.

Examples of the "sulfamoyl group optionally having substituent(s)" include unsubstituted sulfamoyl, N-monosubstituted sulfamoyl group and N,N-disubstituted sulfamoyl group.

The "N-monosubstituted sulfamoyl group" means sulfamoyl group having one substituent on a nitrogen atom. Examples of the substituent are those similar to the substituent of the "N-monosubstituted carbamoyl group".

The "N,N-disubstituted sulfamoyl group" means sulfamoyl group having 2 substituents on a nitrogen atom. Examples of the substituent are those similar to the substituent of the "N,N-disubstituted carbamoyl group".

As the "acyl group derived from sulfonic acid", hydrocarbon group and sulfonyl bonded to each other and the like can be mentioned. Preferred are acyl such as C₁₋₁₀ alkylsulfonyl, C₂₋₆ alkenylsulfonyl, C₂₋₆ alkynylsulfonyl, C₃₋₉ cycloalkylsulfonyl, C₃₋₉ cycloalkenylsulfonyl, C₆₋₁₄ arylsulfonyl, C₇₋₁₀ aralkylsulfonyl and the like. As the C₁₋₁₀ alkyl here, for example, methyl, ethyl, propyl, isopropyl, butyl, isobutyl, t-butyl, pentyl, hexyl, heptyl, octyl, and the like can be mentioned. As the C₂₋₆ alkenyl, for example, vinyl, allyl, 1-propenyl, isopropenyl, 2-butenyl, 3-butenyl, 2-hexenyl, and the like can be mentioned. As the C₂₋₆ alkynyl, for example, ethynyl, 2-propynyl, 2-butynyl, 5-hexynyl, and the like can be mentioned. As the C₃₋₉ cycloalkyl, for example, cyclopropyl, cyclobutyl, cyclopentyl, cyclohexyl, cyclooctyl, and the like can be mentioned. As the C₃₋₉ cycloalkenyl, for example, 1-cyclopenten-1-yl, 2-cyclopenten-1-yl, 3-cyclopenten-1-yl, 3-cyclohexen-1-yl, 3-cycloocten-1-yl, and the like can be mentioned. As the C₆₋₁₄ aryl, phenyl, 1-naphthyl, 2-naphthyl, and the like can be mentioned. As the C₇₋₁₀ aralkylsulfonyl, for example, benzyl, phenethyl, and the like can be mentioned. The hydrocarbon groups bonded to sulfonyl may have substituents, and as the substituent, for example, hydroxy group, optionally substituted amino group [wherein said amino group may have, as a substituent, 1 or 2 from, for example, lower alkyl group (e.g., C₁₋₆ alkyl group such as methyl, ethyl, propyl, isopropyl, butyl, isobutyl, t-butyl, pentyl, hexyl etc., and the like), acyl group (e.g., C₁₋ ₆ alkanoyl such as formyl, acetyl, propionyl, pivaloyl and the like, C₇₋₁₃ arylcarbonyl such as benzoyl and the like, C₁₋₆ alkylsulfonyl such as methylsulfonyl, ethylsulfonyl and the like) and the like], halogen atom (e.g., fluorine, chlorine, bromine, iodine etc.), nitro group, cyano group, lower alkyl group optionally substituted by 1 to 5 halogen atoms (e.g., fluorine, chlorine, bromine, iodine etc.), lower alkoxy group optionally substituted by 1 to 5 halogen atoms (e.g., fluorine, chlorine, bromine, iodine etc.), and the like can be mentioned. As the lower alkyl group, for example, C₁₋₆ alkyl group such as methyl, ethyl, n-propyl, isopropyl, n-butyl, isobutyl, sec-butyl, tert-butyl, pentyl, hexyl and the like, and the like can be mentioned, with particular preference given to methyl, ethyl, and the like. As the lower alkoxy group, for example, C₁₋₆ alkoxy groups such as methoxy, ethoxy, n-propoxy, isopropoxy, n-butoxy, isobutoxy, sec-butoxy, tert-butoxy and the like, and the like can be mentioned, with particular preference given to methoxy, ethoxy, and the like, wherein the same or different 1 or 2 or 3 (preferably 1 or 2) of these substituents is(are) preferably used for the substitution.

As the "cyclic hydrocarbon group" of the "cyclic hydrocarbon group optionally having substituent(s)" represented by R₂, alicyclic hydrocarbon group and aryl group can be mentioned.

Examples of the alicyclic hydrocarbon group include saturated or unsaturated alicyclic hydrocarbon groups, such as cycloalkyl group, cycloalkenyl group, cycloalkanedienyl group and the like. Examples of the cycloalkyl group include C₃₋₉ cycloalkyl (preferably C₃₋₈ cycloalkyl etc.), such as cyclopropyl, cyclobutyl, cyclopentyl, cyclohexyl, cycloheptyl, cyclooctyl, cyclononyl and the like, and condensed rings such as 1-indanyl, 2-indanyl and the like. Examples of the cycloalkenyl group include C₃₋₆ cycloalkenyl groups, such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. Examples of the cycloalkanedienyl group include C₄₋₆ cycloalkanedienyl groups, such as 2,4-cyclopentanedien-1-yl, 2,4-cyclohexanedien-1-yl, 2,5-cyclohexanedien-1-yl and the like, with preference given to C₃₋₈ cycloalkyl group such as cyclohexyl and the like.

Examples of the aryl group include monocyclic or condensed polycyclic aromatic hydrocarbon groups, such as C₆₋₁₄ aryl groups, which are preferably phenyl, naphthyl, anthryl, phenanthryl, acenaphthylenyl, 4-indanyl, 5-indanyl etc., and the like, with particular preference given to phenyl, 1-naphthyl, 2-naphthyl and the like.

As the "substituent" of the "cyclic hydrocarbon group optionally having substituent(s)" represented by R₂, those similar to the "substituent" of the "hydrocarbon group optionally having substituent(s)" mentioned as the "substituent" of the "hydrocarbon group optionally having substituent(s)" represented by R₁ can be mentioned.

As the "heterocyclic group optionally having substituent(s)" represented by R₂, those similar to the "heterocyclic group optionally having substituent(s)" mentioned as the "substituent" of the "hydrocarbon group optionally having substituent(s)" represented by R₁ can be mentioned.

As the halogen atom represented by R₃, for example, fluorine, chlorine, bromine, iodine and the like can be mentioned.

As the "carbamoyl group optionally having substituent(s)", "sulfamoyl group optionally having substituent(s)" and "acyl group derived from sulfonic acid" represented by R₃, those similar to the "carbamoyl group optionally having substituent(s)", "sulfamoyl group optionally having substituent(s)" and "acyl group derived from sulfonic acid" represented by R₁ can be respectively mentioned.

As the "C₁₋₄ alkyl group" of the "C₁₋₄ alkyl group optionally having substituent(s)" represented by R₃, for example, methyl, ethyl, n-propyl, isopropyl, n-butyl, isobutyl, tert-butyl and the like can be mentioned. As the "C₁₋₄ alkoxy group" of the "a C₁₋₄ alkoxy group optionally having substituent(s)" represented by R₃, for example, methoxy, ethoxy, propoxy, n-butoxy, isobutoxy, tert-butoxy and the like can be mentioned.

As the "substituent" of the "C₁₋₄ alkyl group optionally having substituent(s)", "C₁₋₄ alkoxy group optionally having substituent(s)" represented by R₃, those similar to the "substituent" of the "hydrocarbon group optionally having substituent(s)", which are the "substituent" of the hydrocarbon group optionally having substituent(s) represented by R₁ can be mentioned.

As the substituent of the "amino group optionally having substituent(s)" represented by R₃, for example, lower alkyl group (e.g., C₁₋₆ alkyl group such as methyl, ethyl, propyl, isopropyl, butyl, isobutyl, t-butyl, pentyl, hexyl etc., and the like), acyl group derived from carboxylic acid (e.g., C₁₋₆ alkanoyl such as formyl, acetyl, propionyl, pivaloyl and the like), C₇₋₁₅ arylcarbonyl such as benzoyl etc., and the like), acyl group derived from sulfonic acid (e.g., C₁₋₆ alkylsulfonyl such as methylsulfonyl, ethylsulfonyl and the like), optionally halogenated C₁₋₆ alkoxy-carbonyl (e.g., trifluoromethoxycarbonyl, 2,2,2-trifluoroethoxycarbonyl, trichloromethoxycarbonyl, 2,2,2-trichloroethoxycarbonyl etc.), and the like can be mentioned. The "amino group" of the "amino group optionally having substituent(s)" may be substituted by optionally substituted imidoyl group (e.g., C₁₋₆ alkylimidoyl, formylimidoyl, amidino etc.) and the like and 2 substituents may form a cyclic amino group together with nitrogen atom. As the cyclic amino group in such cases, for example, a 3- to 8-membered (preferably 5- or 6-membered) cyclic amino group such as 1-azetidinyl, 1-pyrrolidinyl, 1-piperidinyl, 4-morpholinyl, 1-piperazinyl and 1-piperazinyl optionally having, at the 4-position, lower alkyl group (e.g., C₁₋₆ alkyl group such as methyl, ethyl, propyl, isopropyl, butyl, t-butyl, pentyl, hexyl etc., and the like), aralkyl group (e.g., C₇₋₁₀ aralkyl group such as benzyl, phenethyl etc. and the like), aryl group (e.g., C₆₋₁₀ aryl group such as phenyl, 1-naphthyl, 2-naphthyl etc., and the like), and the like, and the like can be mentioned.

Examples of the salt of the compound of the formula (I) or (II) of the present invention include acid addition salt, such as inorganic acid salts (e.g., hydrochloride, sulfate, hydrobromate, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like. The compound may form salts with a base (e.g., alkali metal salts such as potassium salt, sodium salt, lithium salt etc., alkaline earth metal salts, such as calcium salt, magnesium salt etc. and salts with organic base such as ammonium salt, trimethylamine salt, triethylamine salt, tert-butyldimethylamine salt, dibenzylmethylamine salt, benzyldimethylamine salt, N,N-dimethylaniline salt, pyridine salt, quinoline salt etc).

The compound of the formula (I) or (II) and a salt thereof may be a hydrate, all of which including salts and hydrates, are to be referred to as compound (I) or (II) in the following.

The prodrug of the compound (I) or (II) means a compound that is converted to compound (I) or compound (II) having CCR5 inhibitory action in the body by reaction with an enzyme, gastric acid and the like.

Examples of the prodrug of compound (I) or (II) when the compound (I) or (II) has an amino group include compounds wherein the amino group is acylated, alkylated or phosphorated (e.g., compound wherein the amino group of compound (I) or (II) is eicosanoylated, alanylated, pentylaminocarbonylated, (5-methyl-2-oxo-1, 3-dioxolen-4-yl)methoxycarbonylated, tetrahydrofuranylated, pyrrolidylmethylated, pivaloyloxymethylated, acetoxymethylated, tert-butylated etc.); when compound (I) or (II) has a hydroxy group, a compound wherein the hydroxy group is acylated, alkylated, phosphorated or borated [e.g., compound wherein the hydroxy group of compound (I) or (II) is acetylated, palmitoylated, propanoylated, pivaloylated, succinylated, fumarylated, alanylated, dimethylaminomethylcarbonylated etc.]; when compound (I) or (II) has a carboxyl-group, a compound wherein the carboxyl group is esterified, amidated (e.g., carboxyl group of compound (I) or (II) ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methylamidated etc.); and the like. These compounds can be produced by a method known *per se.*

The prodrug of compound (I) or (II) may be of a kind that changes to compound (I) or (II) under physiological conditions, as described in *Iyakuhin no Kaihatsu,* vol. 7, Molecular Design pp. 163-198, Hirokawa Shoten (1990).

The prodrug of compound (I) or (II) may be as it is or a pharmacologically acceptable salt. Examples of such salt include, when the prodrug of compound (I) or (II) has an acidic group, such as carboxyl group etc., salts with inorganic base (e.g., alkali metal such as sodium, potassium etc., alkaline earth metal such as calcium, magnesium etc., transition metal such as zinc, iron, copper etc. etc.), salts with organic base (e.g., organic amines such as trimethylamine, triethylamine, pyridine, picoline, ethanolamine, diethanolamine, triethanolamine, di cyclohexylamine, N,N'-dibenzylethylenediamine etc., basic amino acids such as arginine, lysine, ornithine etc. etc.), and the like.

When the prodrug of compound (I) or (II) has a basic group, such as amino group and the like, the salt is exemplified by salts with inorganic acid and organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), salts with acidic amino acid, such as aspartic acid, glutamic acid etc., and the like.

The prodrug of compound (I) or (II) may be a hydrate or a non-hydrate.

While it has one or more asymmetric carbon(s) in a molecule, both an R configuration compound and an S configuration compound due to the asymmetric carbons are encompassed in the present invention.

In the present specification, the "lower" as represented by the lower alkyl group, lower alkoxy group and the like means chain, branched or cyclic carbon chain having 1 to 6 carbon atoms, unless particularly specified.

The compounds of the formulas (III), (IV), (V-1), (V-2) and (VI), a compound having a basic group or an acidic group can form a salt with an acid addition salt or a salt with a base. The salts with these acid addition salts and bases are exemplified by those recited with regard to the aforementioned compound (I). In the following, the compounds of the respective formulas, inclusive of salts thereof, are to be briefly referred to as a compound (symbol of the formula). For example, a compound of the formula (III) and a salt thereof are simply referred to as compound (III).

### production method I-1

As shown in the following formulas, compound (IV) and compound (III) can be reacted to produce compound (I). 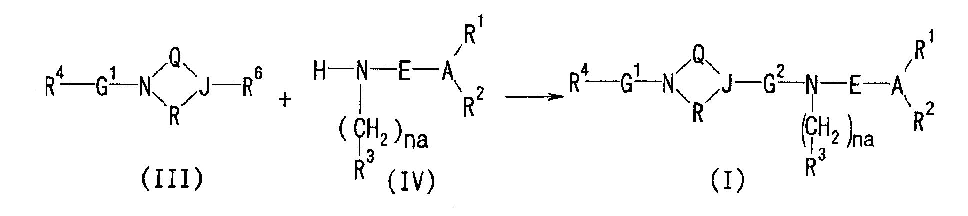 wherein R⁶ is a carboxy group, a sulfonic acid group, a salt thereof or a reactive derivative thereof and other symbols are as defined above.

As the salts of carboxy group or sulfonic acid group represented by R⁶, for example, salts with alkali metal such as sodium, potassium, lithium and the like, salts with alkaline earth metal such as calcium, magnesium, strontium and the like, ammonium salt and the like can be mentioned.

As the reactive derivative of the carboxy group represented by R⁶, for example, reactive derivatives such as acid halide, acid azide, acid anhydride, mixed acid anhydride, active amide, active ester, active thioester, isocyanate and the like are subjected to acylation reaction. As the acid halide, for example, acid chloride, acid bromide and the like can be mentioned; as the mixed acid anhydride, mono C₁₋₆ alkyl carbonate mixed acid anhydride (e.g., mixed acid anhydride of free acid and mono methyl carbonate, mono ethyl carbonate, mono isopropyl carbonate, mono isobutyl carbonate, mono tert-butyl carbonate, mono benzyl carbonate, mono (p-nitrobenzyl) carbonate, mono allyl carbonate and the like), C₁₋₆ aliphatic carboxylic acid mixed acid anhydride (e.g., mixed acid anhydride of free acid and acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid and the like), C₇₋₁₂ aromatic carboxylic acid mixed acid anhydride (e.g., mixed acid anhydride of free acid and benzoic acid, p-toluyl acid, p-chlorobenzoic acid and the like), organic sulfonic acid mixed acid anhydride (e.g., mixed acid anhydride of free acid and methanesulfonic acid, ethanesulfonic acid, benzenesulfonic acid, p-toluenesulfonic acid and the like) and the like can be mentioned; and as the active amide, amide with nitrogen-containing heterocyclic compound [e.g., acid amide of free acid and pyrazole, imidazole, benzotriazole and the like, wherein these nitrogen-containing heterocyclic compounds are optionally substituted by C₁₋₆ alkyl group (e.g., methyl, ethyl etc.), C₁₋₆ alkoxy group (e.g., methoxy, ethoxy etc.), halogen atom (e.g., fluorine, chlorine, bromine etc.), oxo group, thioxo group, C₁₋ ₆ alkylthio group (e.g., methylthio, ethylthio etc.) and the like] and the like can be mentioned.

As the active ester, any can be used as long as it is usable in the field of β-lactam and peptide synthesis for this object, and examples thereof include organic phosphoric acid ester (e.g., diethoxyphosphoric acid ester, diphenoxyphosphoric acid ester and the like), p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester and the like. As the active thioester, ester with aromatic heterocyclic thiol compound [e.g., 2-pyridylthiol ester, 2-benzothiazolylthiol ester and the like, wherein these heterocycles are optionally substituted by C₁₋₆ alkyl group (e.g., methyl, ethyl etc.), C₁₋₆ alkoxy group (e.g., methoxy, ethoxy etc.), halogen atom (e.g., fluorine, chlorine, bromine etc.), C₁₋₆ alkylthio group (e.g., methylthio, ethylthio etc.) and the like] can be mentioned.

As the reactive derivative of the sulfonic acid group represented by R⁶, for example, sulfonyl halide (e.g., sulfonyl chloride, sulfonylbromide and the like), sulfonylazide, acid anhydride thereof and the like can be mentioned.

This reaction generally proceeds in a solvent inert to the reaction. Examples of the solvent include ether solvents (e.g., ethyl ether, diisopropyl ether, dimethoxyethane, tetrahydrofuran, dioxane etc.), halogen solvents (e.g., dichloromethane, dichloroethane, chloroform etc.), aromatic solvents (e.g., toluene, chlorobenzene, xylene etc.), acetonitrile, N,N-dimethylformylamide (DMF), acetone, methyl ethyl ketone, dimethyl sulfoxide (DMSO), water and the like, which are used alone or in combination. Of these, acetonitrile, dichloromethane, chloroform and the like are preferable. This reaction is generally carried out by reacting 1 to 5 equivalents, preferably 1 to 3 equivalents, of compound (III) relative to compound (IV). The reaction temperature is from -20°C to 50°C, preferably 0°C to room temperature, and the reaction time is generally from 5 min to 100 hrs. In this reaction, a co-presence of a base sometimes affords smooth progress of the reaction. As the base, both inorganic bases and organic bases are effective. Examples of the inorganic base include hydroxide, hydride, carbonate, hydrogencarbonate, organic acid salt and the like of alkali metals and alkaline earth metals. Particularly, potassium carbonate, sodium carbonate, sodium hydroxide, potassium hydroxide, sodium bicarbonate and potassium bicarbonate are preferable. As the organic base, tertiary amines such as triethylamine and the like are preferable. Examples of the reactive derivative include acid anhydride, acid halide (e.g., acid chloride and acid bromide), active ester, isocyanate and the like, with preference given to acid halide. The amount of use of the base is generally 1 to 10 equivalents, preferably 1 to 3 equivalents, relative to compound (IV).

In the case of acylation from carboxylic acid, 1 equivalent of compound (IV) is reacted with 1 to 1.5 equivalents of carboxylic acid in an inert solvent (e.g., halogen solvent and acetonitrile) in the presence of 1 to 1.5 equivalents of a dehydrative condensing agent such as dicyclohexylcarbodiimide (DCC) and the like. This reaction generally proceeds at room temperature-where the reaction time is 0.5 to 24 hrs.

In compound (IV) to be used for this method, the divalent chain hydrocarbon group optionally substituted by a group other than oxo group, as represented by E, is a group of the formula 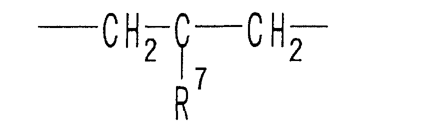 wherein R⁷ is a substituent other than an oxo group, for example, the compound can be produced by a method described in Synthetic Comm., 1991, 20, 3167-3180. That is, utilizing the addition reaction of amines or amides to unsaturated bond, the following method is employed for the production. 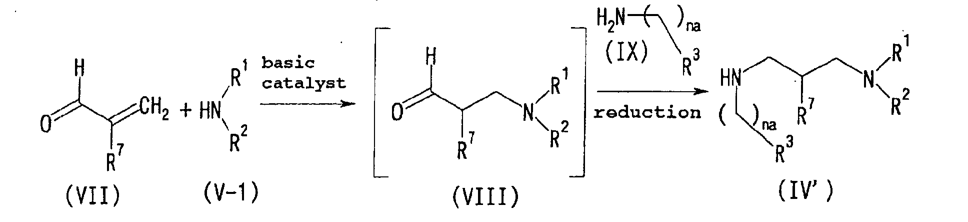 wherein each symbol is as defined above.

The substituent other than oxo group represented by R⁷ means the substituent other than oxo group of the divalent chain hydrocarbon group optionally having substituent(s) other than oxo group, as represented by E.

The compound can be obtained by reacting acrolein derivative (VII) and compound (V-1) and then reacting the obtained product with compound (IX) under reducing conditions. The reaction between compound (VII) and compound (V-1) is generally carried out in a solvent inert to the reaction in the presence of a base. Examples of the base include 1) strong base such as hydride of alkali metal or alkaline earth metal (e.g., lithium hydride, sodium hydride, potassium hydride, calcium hydride etc.), amide of alkali-metal or alkaline earth metal (e.g., lithiumamide, sodiumamide, lithium diisopropylamide, lithium dicyclohexylamide, lithium hexahexamethylsilazide, sodium hexahexamethylsilazide, potassium hexahexamethylsilazide etc.), lower alkoxide of alkali metal or alkaline earth metal (e.g., sodium methoxide, sodium ethoxide, potassium t-butoxide etc.) and the like, 2) inorganic base such as hydroxide of alkali metal or alkaline earth metal (e.g., sodium hydroxide, potassium hydroxide, lithium hydroxide, barium hydroxide etc.), carbonate of alkali metal or alkaline earth metal (e.g., sodium carbonate, potassium carbonate, cesium carbonate etc.), hydrogencarbonate of alkali metal or alkaline earth metal (e.g., sodium hydrogencarbonate, potassium hydrogencarbonate etc.) and the like, 3) organic base and the like such as amines [e.g., triethylamine, diisopropylethylamine, N-methylmorpholine, dimethylaminopyridine, DBU (1,8-diazabicyclo[5.4.0]-7-undecene), DBN (1,5-diazabicyclo[4.3.0]-non-5-ene) etc.] and basic heterocyclic compound (e.g., pyridine, imidazole, 2,6-lutidine etc.), basic compound and the like. Examples of the solvent include those recited for the reaction of the aforementioned compound (IV) and compound (III), which can be used alone or in combination or can be used. By this reaction, compound (VIII) is obtained.

Examples of the reducing agent to be used for the reaction of compound (VIII) and compound (IX) include sodium borohydride, lithium borohydride, sodium cyanoborohydride and the like. These reducing agents are used in an amount of generally 1 to 10 equivalents, preferably 1 to 4 equivalents, relative to compound (VIII). The reaction temperature is from -20°C to -50°C, preferably 0°C to room temperature and the reaction time is 0.5 - 24 hrs.

The catalytic reduction is conducted by a reaction with a catalytic amount of a metal catalyst, such as Raney Nickel, platinum oxide, metal palladium, palladium-carbon etc. in an inert solvent (e.g., alcohol solvent such as methanol, ethanol, isopropanol, t-butanol etc.) at room temperature to 100°C at a hydrogen pressure of 1 atm to 100 atm for 1 to 48 hrs.

The compound (IV) used for this method can be produced by a method described in, for example, method described in Chem. Pharm. Bull. 47(1) 28-36 (1999), JP-A-56-53654 and the like or a method analogous thereto.

The compound (III) to be used for this method can be produced by a method described in, for example, J. Am. Chem. Soc., 1950, 72, 1415., J. Am. Chem. Soc., 1952, 74, 4549, J. Org. Chem., 1956, 21, 1087 and the like or a method analogous thereto.

### Production Method I-2

As shown in the following formulas, compound (VI) and compound (V-1) or (V-2) can be reacted to produce compound (I). 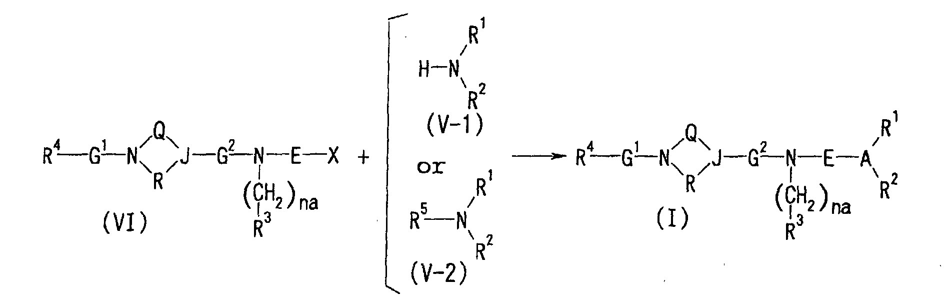 wherein X is a leaving group and other symbols are as defined above.

As the leaving group represented by X, for example, halogen atom (e.g., chlorine atom, bromine atom, iodine atom and the like), alkyl or arylsulfonyloxy group (e.g., methanesulfonyloxy, ethanesulfonyloxy, benzenesulfonyloxy, p-toluenesulfonyloxy and the like) and the like can be mentioned.

This reaction can be carried out according to the method described in, for example, ORGANIC FUNCTIONAL GROUP PREPARATIONS, 2nd printing, ACADEMIC PRESS, INC.

This reaction is generally carried out in a solvent inert to the reaction. Examples of the solvent include alcohol solvents, ether solvents, halogen solvents, aromatic solvents, acetonitrile, N,N-dimethylformamide (DMF), acetone, methyl ethyl ketone, dimethyl sulfoxide (DMSO) and the like, which may be used alone or in combination. Of these, acetonitrile, dimethylformamide, acetone, ethanol and the like are preferable. The reaction temperature is generally from room temperature to 100°C, preferably from room temperature to 50°C and the reaction time is generally from 0.5 to one day. For this reaction, 1 to 3 equivalents of a base is generally added relative to compound (VI), but it is not essential. Examples of the base include the base used for the reaction of the above-mentioned compound (IV) and compound (III).

The compound (VI) used as a starting material for this reaction can be synthesized by a known method using compound (III) as a starting material.

### Production method I-3

Of the compounds (I), a compound wherein E is represented by the formula 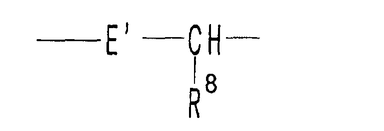 wherein E' is a group E having less one carbon atoms, R⁸ is a hydrogen atom or a hydrocarbon group, can be produced as shown in the following formulas, wherein compound of the formula (X) and compound of the formula (V-1) are reacted under reducing conditions to give the compound. 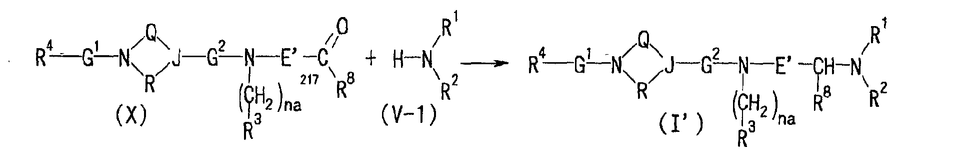 wherein each symbol is as defined above.

The group represented by E' which has less one carbon atoms as compared to E is a divalent chain hydrocarbon group optionally having substituent(s) other than oxo group and has carbon atoms of E less one. Examples of the hydrocarbon group represented by R⁸ include unsubstituted alkyl group, aryl group, cycloalkyl group and cycloalkenyl group from the optionally substituted alkyl group, optionally substituted aryl group, optionally substituted cycloalkyl group and optionally substituted cycloalkenyl group, which have been exemplified as the substituents other than an oxo group of a divalent chain hydrocarbon group optionally having substituent(s) other than oxo group, as represented by E.

This reaction is carried out generally by reacting compound (X) and compound (V-1) in a suitable solvent (e.g., water, alcohol, ether, halogen, acetonitrile, mixed solvent of two or more kinds of these etc.), adding an acidic substance where necessary, such as acetic acid, trifluoroacetic acid and the like, in the presence of a compound (1 - 5 equivalents, preferably 1 - 1.5 equivalents), wherein carbonyl group is added to alkyl group, and a reducing agent. The reducing agent and other conditions are the same as those described for the method of Production Method I-1.

The compound (X) used as a starting material for this reaction can be produced by a known method using compound (III) as a starting material.

### Production method I-4

Of the compounds (I), a compound wherein E is represented by the formula 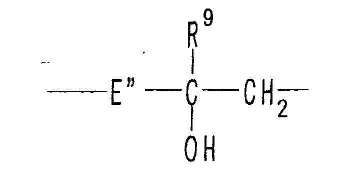 wherein E" is a group E having less two carbon atoms and R⁹ is a hydrocarbon group, can be produced as shown by reacting compound of the formula (XI) and compound of the formula (V-1). 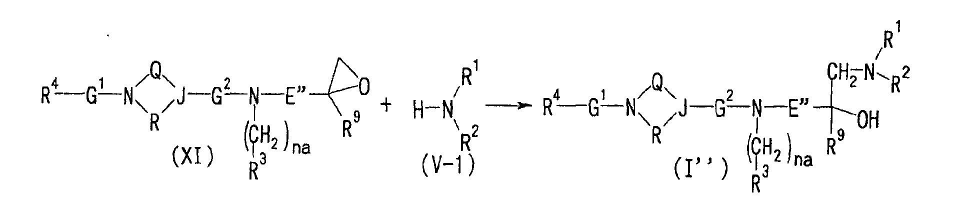 wherein each symbol is as defined above.

The group represented by E" which has less two carbon atoms as compared to E is a divalent chain hydrocarbon group optionally having substituent(s) other than an oxo group and has carbon atoms of E less two. Examples of the hydrocarbon group represented by R⁹ include hydrocarbon groups exemplified for R⁸.

This reaction is carried out in the presence or absence of a solvent. Examples of the solvent include those recited for the reaction of the aforementioned compound (IV) and compound (III). For this reaction, a Lewis acid such as anhydrous zinc chloride, anhydrous aluminum chloride, anhydrous iron (IV) chloride, titanium tetrachloride, tin tetrachloride, cobalt chloride, copper(IV) chloride, boron trifluoride etherate etc. or the aforementioned base can be used as a catalyst to accelerate the reaction. The reaction temperature is generally from -40°C to 180°C.

The compound (XI) used as a starting material for this reaction can be synthesized by a known method using compound (III) as a starting material.

### Production method I-5

The compound (XII) and compound (XIII) are reacted to produce compound (I). 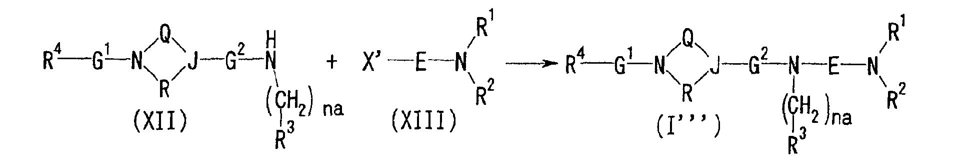 wherein X' is a leaving group and other symbols are as defined above.

Examples of the leaving group represented by X' include those exemplified as the leaving group represented by X. This reaction can be carried out according to the method of Production Method I-2.

The compound (XIII) used as a starting material for this reaction can be produced from compound (V-1) by a known method.

The compound (XII) used as a starting material for this reaction can be synthesized by reacting compound (III) and a compound of the formula H₂N(CH₂)ₙ-R³ according to the method of Production Method I-1.

### Production method I-6

The compound (I) can be produced by reacting compound (XIV) with compound (XV) as shown in the following formulas. 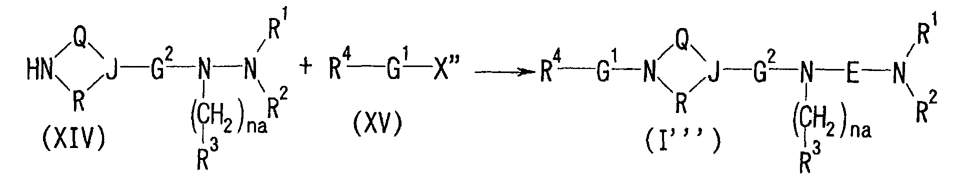 wherein X" is a leaving group, or G¹-X" represents carboxyl group, sulfonic acid group or a reactive derivative thereof, and other symbols are as defined above.

As the reactive derivative of the carboxyl group and sulfonic acid group represented by G¹-X", those similar to the reactive derivative of the carboxyl group and sulfonic acid group represented by R⁶ can be mentioned.

This reaction can be carried out according to the above-mentioned production method I-2. As the leaving group represented by X", those mentioned for the leaving group represented by X can be mentioned.

The compound (II) can be produced by, for example, the method shown in the following, and the like.

In the compounds represented by the following formulas (XVI), (XVII), (XVIII), (XIX), (XXI), (XXII) and (XXIII), the compound having a basic group or acidic group can form a salt with an acid addition salt or a base. As these salts with the acid addition salt and the base, those similar to the salts of the compound represented by the aforementioned formula (XVI) can be mentioned. Hereinafter the compounds represented by respective formulas including salts thereof are abbreviated as compound (symbol of formula). For example, a compound represented by the formula (XVII) and a salt thereof are simply referred to as compound (XVII).

### Production method 2-1

As shown in the following formula, Compound (XVI) is reacted with Compound (XVII) to give Compound (II). 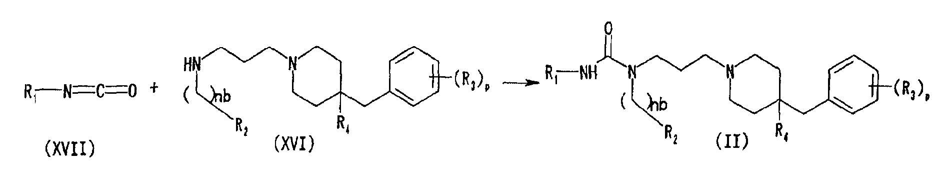 wherein each symbol has the same meaning as defined above.

The reaction is usually carried out in a solvent inert to the reaction. Examples of the solvent include an ether (e.g., ethyl ether, diisopropyl ether, dimethoxy ethane, tetrahydrofuran, dioxane etc.), a halogenated hydrocarbon (e.g., dichloromethane, dicholoroethane, chloroform etc.), an aromatic solvent (e.g., toluene, chlorobenzene, xylene etc.), acetonitrile, N,N-dimethylformamide (DMF), acetone, methylethyl ketone, dimethylsulfoxide (DMSO), water, etc., or a mixed solvent thereof. Among them, acetonitrile, dichloromethane, chloroform, etc. are preferable. The reaction is usually carried out by using 1 to 5 equivalent(s), preferably 1 to 3 equivalents of Compound (XVII) relative to 1 equivalent of Compound (XVI). The reaction temperature ranges from -20°C to 50°C, preferably 0°C to room temperature, and reaction time is usually 5 minutes to 100 hrs. The reaction may smoothly proceed by using a base. As the base, an inorganic base and an organic base can be used effectively. Examples of the inorganic base include a hydroxide, a hydride, a carbonate, a bicarbonate of alkaline metal or alkaline earth metal. Among them, potassium carbonate, sodium carbonate, sodium hydroxide, potassium hydroxide, sodium hydrogencarbonate, potassium hydrogencarbonate are preferable. Examples of the organic base preferably include a tertiary amine such as triethylamine.

Compound (XVI) can be produced, for example, by a method described in Synthetic Comm., 1991, 20, 3167-3180. That is, the above compound can be produced by the following method by applying an addition reaction of amines or amides to unsaturated bond. 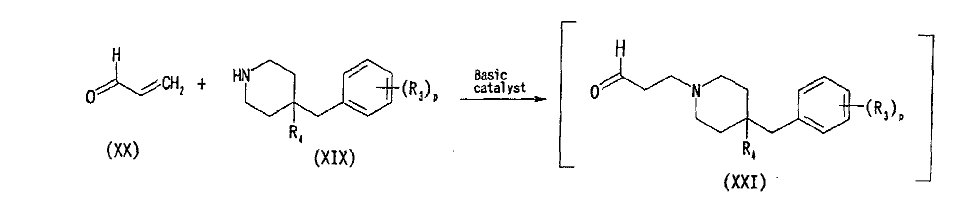 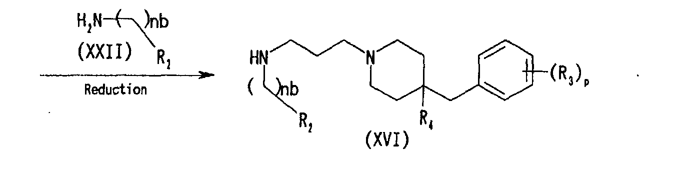 wherein each symbol is as defined above.

The compound (XVI) can be produced by reacting acrolein (XX) with Compound (XIX), followed by reacting the resulting compound with Compound (XXII) under a condition of reduction. The reaction of Compound (XX) with Compound (XIX) is usually carried out in a solvent inert to the reaction in the presence of a base. Examples of the base include 1) a strong base and the like, such as hydride of alkali metal or alkaline earth metal (e.g., lithium hydride, sodium hydride, potassium hydride, calcium hydride etc.), an amide of an alkali metal or an alkaline earth metal (e.g., lithium amide, sodium amide, lithium diisopropylamide, lithium dicyclohexylamide, lithium hexamethyldisilazide, sodium hexamethyldisilazide, potassium hexamethyldisilazide etc.), a lower alkoxide of alkali metal or alkaline earth metal (e.g., sodium methoxide, sodium ethoxide, potassium t-butoxide etc.), etc., 2) an inorganic base such as a hydroxide of an alkali metal or an alkaline earth metal (e.g., sodium hydroxide, potassium hydroxide, lithium hydroxide, barium hydroxide etc.), a carbonate of an alkali metal or an alkaline earth metal (e.g., sodium carbonate, potassium carbonate, cesium carbonate etc.), a bicarbonate of alkali metal or alkaline earth metal (e.g., sodium hydrogencarbonate, potassium hydrogencarbonate etc.), etc., 3) an organic base, etc., such an amine as triethylamine, diisopropylethylamine, N-methylmorpholine, dimethylaminopyridine, DBU (1,8-diazabicyclo[5.4.0]-7-undecene), DBN (1,5-diazabicyclo[4.3.0]non-5-ene), etc., and such basic heterocyclic compound, etc., as pyridine, imidazole, 2,6-lutidine, etc. Examples of the solvent include those mentioned in the reaction of Compound (XVI) with Compound (XVII). These solvents can be used solely or in combination. Compound (XXI) can be obtained in the reaction.

Examples of the reducing agent for the reaction of Compound (XXI) with Compound (XXII) include sodium borohydride, lithium borohydride, sodium cyanoborohydride, sodium triacetoxyborohydride, etc. The used amount of the reducing agent is usually in the range of 1 to 10 equivalents, preferably in the range of 1 to 4 equivalents relative to 1 equivalent of Compound (XXI). The reaction temperature ranges -20°C to 50°C, preferably 0°C to room temperature, and reaction time is 0.5 to 24 hours.

Catalytic reduction reaction is carried out in the presence of a catalytic amount of a metal catalyst such as Raney nickel, platinum oxide, metallic palladium, palladium-carbon, etc., in an inert solvent (e.g., an alcohol such as methanol, ethanol, isopropanol, t-butanol etc.), at room temperature to 100°C, under a hydrogen pressure of 1 to 100 atm for 1 to 48 hours.

### Production method 2-2

Compound (II) can be produced by reacting Compound (XVIII) with Compound (XIX) as shown below. 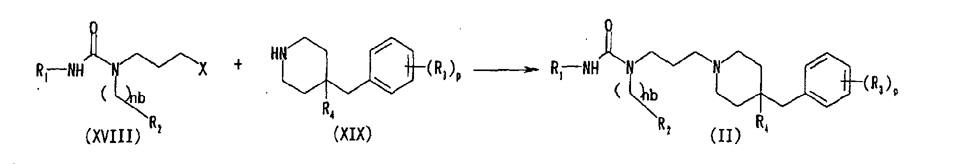 wherein each symbol is as defined above.

The reaction can be carried out by a manner similar to that described in Organic Functional Group Preparations 2nd ed., (Academic Press, Inc.).

The reaction is usually carried out in a solvent inert to the reaction. Examples of the solvent include an alcohol, an ether, a halogen solvent, an aromatic solvent, acetonitrile, N,N-dimethylformamide (DMF), acetone, methylethyl ketone, dimethylsulfoxide (DMSO), etc. These solvents can be used solely or in combination. Among them, acetonitrile, dimethylformamide, acetone, ethanol, etc., are preferable. The reaction temperature ranges usually from room temperature to 100°C, preferably from room temperature to 50°C, and the reaction time is usually 0.5 to 1 day. In this reaction, a base is usually added in an amount of 1 to 3 equivalents relative to 1 equivalent of Compound (XVIII), but it is not essential. Examples of the base include those mentioned in the reaction of Compound (XVI) with Compound (XVII).

Compound (XVIII) used as a starting material in the reaction can be produced from Compound (XVII) by a known conventional method.

### Production method 2-3

Compound (II) can be produced by reacting a compound of the formula (XXIII) with a compound of the formula (XIX) under a reduction condition as shown below. 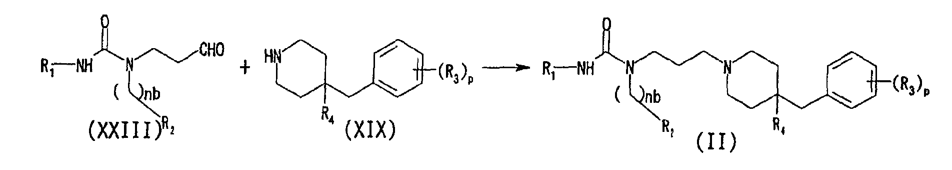 wherein each symbol is as defined above.

The reaction is carried out by reacting Compound (XXIII) with Compound (XIX) in an appropriate solvent (e.g., water, an alcohol, an ether, a halogenated solvent, acetonitrile, or a mixed solvent of two or more of these solvents etc.), if necessary, by the addition of acidic substance such as acetic acid, trifluoroacetic acid, etc., in the presence of 1 to 5 equivalents, preferably 1 to 1.5 equivalent of a reducing agent. The reducing agent and the reaction condition mentioned in Production 2-1 can be applied for this reaction. Compound (XXIII) used as a starting material in the reaction can be produced from Compound (XVII) by a known conventional method.

The compounds (I) and (II) of the present invention have a CCR antagonistic action, particularly potent CCR5 antagonistic action and are low toxic.

The "piperidine compound having CCR5 antagonistic action" to be used in the present invention, a compound represented by the formula (I) and a salt thereof, a compound represented by the formula (II) and a salt thereof, mean piperidine ring-containing compound, and the like from among the compounds having CCR5 antagonistic action, which are described in WO99/04794, WO99/38514 and WO00/35451; and the "compound having CCR5 antagonistic action" includes a compound represented by the formula (I) and a salt thereof and a compound represented by the formula (II) and a salt thereof and the above-mentioned "a piperidine compound having CCR5 antagonistic action".

As the compound having CCR5 antagonistic action to be used in the present invention, N-(3,4-dichlorophenyl)-1-(methylsulfonyl)-N-{3-[4-({4-[(methylsulfonyl)amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl) -4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl -N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide and salts thereof are preferable.

The content of the compound having CCR5 antagonistic action in the oral pharmaceutical composition of the present invention is 0.01-90 W/W%, preferably 0.1-50 W/W%, more preferably 1-30 W/W%.

As the amphiphilic substance to be used in the present invention, a combination of one or two kinds selected from sorbitan fatty acid esters such as sorbitan monolaurate, sorbitan monopalmitate, sorbitan monostearate, sorbitan trioleate, sorbitan monooleate and the like; saturated polyglycolated glycerides such as Gelucire (35/10, 44/14, 46/07, 50/13, 53/10), LABRASOL, LABRAFIL ISOSTEARIQUE, LABRAFAC CH10 (Gattefose) and the like, unsaturated polyglycolated glycerides such as LABRAFIL WL 2609BS (Gattefose), LABRAFIL M 1944CS (Gattefose), LABRAFIL M 2125CS (Gattefose) and the like; polyglycerine fatty acid esters such as tetraglycerine monooleate (MO-310; Sakamoto Yakuhin Kogyo Co., Ltd.), tetraglycerine monolaurate (ML-310; Sakamoto Yakuhin Kogyo Co., Ltd.), tetraglycerine monocaprylate (MCA-310; Sakamoto Yakuhin Kogyo Co., Ltd.), hexaglycerine monooleate (MO-500; Sakamoto Yakuhin Kogyo Co., Ltd.), hexaglycerine monolaurate (ML-500; Sakamoto Yakuhin Kogyo Co., Ltd.), hexaglycerine monocaprylate (MCA-500; Sakamoto Yakuhin Kogyo Co., Ltd.), decaglycerine monooleate (MO-750; Sakamoto Yakuhin Kogyo Co., Ltd.), decaglycerine monolaurate (ML-750; Sakamoto Yakuhin Kogyo Co., Ltd.), decaglycerine monocaprylate (MCA-750; Sakamoto Yakuhin Kogyo Co., Ltd.), tetraglycerine monostearate (MS-310; Sakamoto Yakuhin Kogyo Co., Ltd.), hexaglycerine monostearate (MS-500; Sakamoto Yakuhin Kogyo Co., Ltd.), hexaglycerine sesquistearate (SS-500; Sakamoto Yakuhin Kogyo Co., Ltd.), decaglycerine monostearate (MS-750; Sakamoto Yakuhin Kogyo Co., Ltd.), decaglycerine sesquioleate (SO-750; Sakamoto Yakuhin Kogyo Co., Ltd.), PLUROL OLEIQUE CC 497 (Gattefose) and the like; water-soluble vitamin E derivatives such as D-α-tocopherol polyethylene glycol 1000 succinate (Vitamin E TPGS, Eastman Chemical) and the like; polyoxyethylenepolypropylene glycols such as Poloxamer 188 (Pluronic F-68, BASF Corporation) and the like; mono fatty acid esters of polyoxyethylene(20)sorbitan such as Tween 20, 40, 60, 80 and the like; polyoxyethylene castor oil derivatives such as Cremophor EL (BASF Corporation), Cremophor RH40 (BASF Corporation) and the like; propylene glycol laurates such as LAUROGLYCOL FCC (Gattefose) and the like and the like can be mentioned. Of these, a combination of one or two kinds selected from water-soluble vitamin E derivatives, saturated polyglycolated glycerides, unsaturated polyglycolated glycerides, sorbitan fatty acid esters, polyglycerine fatty acid esters, polyoxyethylenepolypropylene glycols, polyoxyethylene castor oil derivatives and propylene glycol laurates is preferable, a combination of one or two kinds selected from self-emulsifying type surfactants such as water-soluble vitamin E derivatives, saturated polyglycolated glycerides, unsaturated polyglycolated glycerides and the like is more preferable, wherein water-soluble vitamin E derivatives are still more preferable, and D-α-tocopherol polyethylene glycol 1000 succinate is most preferable. By the use of such a self-emulsifying type surfactant, a composition having a self-emulsifying action to spontaneously produce a solubilizing liquid (micelle, microemulsion, emulsion and the like) by stirring in water or upon contact with a digestive juice in the living organism can be obtained.

When Gelucire 50/13 (Gattefose) or Gelucire 53/10 (Gattefose) is used as an amphiphilic substance, sustained-release property can be imparted.

The amount of the amphiphilic substance in the oral pharmaceutical composition of the present invention is generally 10-99.99 W/W%, preferably 50-99.9 W/W%, more preferably 70-99 W/W%.

The oral pharmaceutical composition of the present invention may consist of the above-mentioned constituent component, namely, compound (I) or (II) and an amphiphilic substance alone, but a pharmaceutically acceptable additive may be added as appropriate. For example, as an additive (oral absorption acceleration aid) to aid the promotion of oral absorption of the composition of the present invention, organic acids such as acetic acid, propionic acid, capric acid, stearic acid, sorbic acid, oleic acid, benzoic acid, lactic acid, gluconic acid, dehydroacetic acid, erythorbic acid, deoxycholic acid, cholic acid, lauryl sulfuric acid, taurine, salicylic acid, myristic acid, palmitic acid, linoleic acid, lauric acid, capric acid, caproic acid, capric acid and the like (preferably salicylic acid, deoxycholic acid, myristic acid and the like), alkali metal salts (e.g., sodium salt, potassium salt and the like), alkaline earth metal salts (e.g., calcium salt, magnesium salt and the like) and the like may be added as appropriate. Particularly, absorption acceleration aids such as sodium salicylate, sodium deoxycholate, sodium myristate and the like are preferably used.

The absorption acceleration aid is generally added in a proportion of 0-90 W/W%, preferably 0-50 W/W%, more preferably 0-30 W/W to the oral pharmaceutical composition of the present invention.

Where necessary, preparation additives such as dissolution aid, lipid, preservative, antioxidant, coloring agent, sweetening agent and the like can be used.

As the dissolution aid, alcohol, propylene glycol, polyethylene glycol, macrogol, D-mannitol, benzyl benzoate, triaminomethane, cholesterol, triethanolamine, sodium lauryl sulfate, lauryl aminopropionate, lactic acid, caproic acid, caprylic acid, capric acid, oleic acid, linoleic acid, lecithin, benzalkonium chloride, benzethonium chloride, glycerine monostearate, polyvinyl alcohol, carboxymethyl cellulose sodium, methyl cellulose, hydroxymethyl cellulose, hydroxyethyl cellulose, hydroxypropyl cellulose, polyacrylic acid derivative or a salt thereof (carboxyvinyl polymer and the like), tannic acid, propyl gallate can be mentioned.

When a dissolution aid is to be used, the amount of the dissolution aid to be contained in the oral pharmaceutical composition of the present invention is generally 0-50 W/W%, preferably 0-40 W/W%, more preferably 0-20 W/W%.

As the lipid, fats and oils such as LABRAFAC LIPOPHILE, LABRAFAC CC, Gelucire33/01, Gelucire39/01, Gelucire43/01, LABRAFAC PG, olive oil, sesame oil, soybean oil, corn oil, rapeseed oil, castor oil, palm oil, eucalyptus oil and the like; middle chain fatty acid triglycerides such as Miglyol 812 and the like; polyglycerine fatty acid esters such as tetraglycerine polyricinolate (CR-310, Sakamoto Yakuhin Kogyo Co., Ltd.), hexaglycerine polyricinolate (CR-500, Sakamoto Yakuhin Kogyo Co., Ltd.), condensation polyricinolate (CRS-ED, CRS-75, Sakamoto Yakuhin Kogyo Co., Ltd.), tetraglycerine fatty acid ester (THL-3, THL-15, Sakamoto Yakuhin Kogyo Co., Ltd.) and the like; Sefsol 228, yolk lecithin and the like can be mentioned.

When a lipid is to be used, the amount of the lipid to be contained in the oral pharmaceutical composition of the present invention is generally 0-50 W/W%, preferably 0-30 W/W%, more preferably 0-20 W/W%.

Preferable examples of the preservative include p-oxybenzoic acid esters, chlorobutanol, benzyl alcohol, phenethyl alcohol, dehydroacetic acid, sorbic acid and the like. Preferable examples of the antioxidant include sulfite, ascorbic acid, tocopherol and the like.

When a preservative is to be used, the amount of the preservative to be contained in the oral pharmaceutical composition of the present invention is generally 0-10 W/W%, preferably 0-8 W/W%, more preferably 0-5 W/W%.

The oral pharmaceutical composition of the present invention can be produced by, for example, adding a compound having CCR5 antagonistic action to at least one kind of amphiphilic substance or a mixture thereof with an additive (hereinafter these are referred to as an amphiphilic substance-containing carrier, in a liquid state by heating as necessary) and stirring the mixture to dissolve or disperse the compound having CCR5 antagonistic action in a homogenous micro-state. The stirring can be performed using, for example, a stirrer or emulsifier generally used for the production of food or pharmaceutical products, such as propeller stirrer, homomixer, sonicator, vortex stirrer, Gaulin homogenizer, microfluidizer and the like.

The oral pharmaceutical composition of the present invention can be also produced by dissolving or dispersing a compound having CCR5 antagonistic action in a homogenous micro-state in an amphiphilic substance-containing carrier melted at a temperature higher than the melting point (preferably a temperature 10°C or more higher than the melting point; more preferably a temperature 20°C or more higher than the melting point), followed by cooling. Alternatively, the oral pharmaceutical composition of the present invention can be produced from a dispersion wherein a compound having CCR5 antagonistic action is dispersed in water or an aqueous solution wherein the compound is dissolved by heating and an amphiphilic substance-containing carrier.

The oral pharmaceutical composition of the present invention may be any of a liquid state, a semi-solid state (e.g., intermediate state between a liquid state and a solid state such as a paste and the like) and a solid state at 15°C to 25°C, with preference given to a liquid state or a semi-solid state.

The oral pharmaceutical composition of the present invention can be produced by dissolving or dispersing a compound having CCR5 antagonistic action in an amphiphilic substance-containing carrier, but may be used as an emulsion formed by appropriately adding water. In this case, water to be added may be contained in any of a compound having CCR5 antagonistic action and an amphiphilic substance-containing carrier. The amphiphilic substance-containing carrier (preferably self-emulsifying type surfactant) is preferably used.

Moreover, the composition of the present invention can be used upon adsorption onto or mixing with a pharmaceutically acceptable powder additive. As such powder additive, for example, lactose, crystalline cellulose, titanium oxide, talc, synthesis aluminum silicate, crosscarmelose sodium, starch, calcium carbonate, hydroxypropylcellulose, sodium carboxymethyl starch, crosspovidone and the like can be mentioned. The weight ratio of the added powder to the composition is about 0.01-100 W/W%, preferably about 0.1-10 W/W%, more preferably about 1-3 W/W%.

The dosage form is not particularly limited as long as it can be administered orally, and liquid, elixir, capsule, granule, suspension, emulsion, powder, tablet, syrup and the like are examples thereof, with preference given to capsule.

In addition, a capsule preparation can be produced by filling in a hard capsule or soft capsule such as gelatin capsule, HPMC capsule and the like, using a conventional liquid, semi-solid capsule filling machine.

When the oral pharmaceutical composition of the present invention is orally administered, the availability of a compound having CCR5 antagonistic action becomes generally 1.2 times or more higher than a conventional preparation composition (e.g., a powder of the compound having CCR5 antagonistic action itself, a solution wherein the powder is dissolved or dispersed in water or methyl cellulose or a tablet produced using excipient, lubricant and the like conventionally used for tablet production and the like), and the compound having CCR5 antagonistic action shows less inconsistent absorption.

The "compound having CCR5 antagonistic action" or a salt thereof to be used in the present invention may be used along with other prophylactic or therapeutic agent of HIV infectious disease (particularly, prophylactic or therapeutic agent of AIDS) in combination.

Specific examples of other agents for the prophylaxis or treatment of HIV infectious diseases, which are used in combination with the "compound having CCR5 antagonism" of the present invention, include nucleoside reverse transcriptase inhibitors such as zidovudine, didanosine, zalcitabine, lamivudine, stavudine, abacavir, adefovir, adefovir dipivoxil, fozivudine tidoxil and the like; non-nucleoside reverse transcriptase inhibitors such as nevirapine, delavirdine, efavirenz, loviride, immunocal, oltipraz and the like, inclusive of pharmaceutical agents having antioxidant action such as immunocal, oltipraz and the like; protease inhibitors such as saquinavir, ritonavir, indinavir, nelfinavir, amprenavir, palinavir, lasinavir and the like; and the like.

As the nucleoside reverse transcriptase inhibitors, zidovudine, didanosine, zalcitabine, lamivudine, stavudine, abacavir and the like are preferable, as the non-nucleoside reverse transcriptase inhibitors, nevirapine, delavirdine, efavirenz and the like are preferable, and as the protease inhibitor, saquinavir, ritonavir, indinavir, nelfinavir, amprenavir and the like are preferable.

The compounds (I) and (II) can be used in combination with the above-mentioned protease inhibitor, nucleic acid reverse transcriptase inhibitor and the like, as well as, for example, antagonist of CXCR4, which is a second receptor of T-cell tropic HIV-1 (e.g., AMD.8664 and the like), CD4 antagonist, entry inhibitor (e.g., T-20, FP-21399 and the like) that acts on the surface antigen of HIV-1 to inhibit invasion of virus into a host cell, integrase inhibitor that inhibits incorporation of virus DNA into a host chromosome, Tat inhibitor that acts on Tat, which is a transcription factor of HIV-1 to inhibit transcription of virus DNA to mRNA and HIV-1 vaccines.

Since the compounds (I) and (II) have CCR antagonistic action, particularly potent CCR5 antagonistic action, and have low toxicity, the pharmaceutical composition of the present invention can be used as a prophylactic or therapeutic agent of various HIV infectious diseases in human, such as a prophylactic or therapeutic agent of AIDS, an agent for suppressing progress of AIDS, a prophylactic or therapeutic agent of multiple sclerosis, a prophylactic or therapeutic agent of graft versus host reaction, a prophylactic or therapeutic agent of chronic rheumatoid arthritis and the like.

While the daily dose of the compound (I) varies depending on the condition and body weight of patients and administration route, it is about 5 to 1000 mg, preferably about 10 to 600 mg, more preferably about 10 to 300-mg, particularly preferably about 15 to 150 mg, in the amount of the active ingredient [CCR5 antagonist] in the case of oral administration to an adult (body weight 50 Kg), which is administered once or in two or three divided portions a day.

When the CCR5 antagonist and a reverse transcriptase inhibitor and/or a protease inhibitor are used in combination, the dose of the reverse transcriptase inhibitor or the protease inhibitor is appropriately determined within the range of not less than about 1/200 to 1/2 and not more than about 2 to 3 times the typical dose. Moreover, when two or more kinds of pharmaceutical agents are used in combination, and when one pharmaceutical agent affects metabolism of a different pharmaceutical agent, the dose of each pharmaceutical agent is adjusted as appropriate. In general, a dose for a single administration of each pharmaceutical agent is employed.

The present invention is explained in more detail in the following by referring to Examples and Experimental Examples. However, these are mere examples and do not limit the present invention in any way. In the present specification, "room temperature" means 5°C to 35°C.

### Examples

### Example 1

To Vitamin E TPGS (970 mg) melted at a temperature 20°C or more higher than the melting point was added N-(3,4-dichlorophenyl)-1-(methylsulfonyl)-N-(3- {4-[4-(methylsulfonyl)benzyl]-1-piperidinyl}propyl)-4-piperidinecarboxamide [hereinafter to be referred to as compound A] (30 mg) with stirring and the mixture was thoroughly stirred to allow complete dissolution in Vitamin E TPGS, whereby a composition was obtained, which was then cooled to room temperature.

### Example 2

To Vitamin E TPGS (270 mg) melted at a temperature 20°C or more higher than the melting point was added compound A (30 mg) with stirring and the mixture was thoroughly stirred to allow complete dissolution in Vitamin E TPGS, whereby a composition was obtained, which was then cooled to room temperature.

### Example 3

To Vitamin E TPGS (240 mg) melted at a temperature 20°C or more higher than the melting point was added compound A (60 mg) with stirring and the mixture was thoroughly stirred to allow complete dissolution in Vitamin E TPGS, whereby a composition was obtained, which was then cooled to room temperature.

### Example 4

To a solution of LABRASOL, LABRAFIL WL 2609BS and TRANSCUTOL homogenously mixed at a weight ratio of 45/40/15 [hereinafter to be referred to as L/L/T (45/40/15)] (970 mg) was added compound A (30 mg) with stirring and the mixture was thoroughly stirred to allow complete dissolution in L/L/T (45/40/15), whereby a composition was obtained.

### Example 5

To Vitamin E TPGS (108 mg) melted at a temperature 20°C or more higher than the melting point was added N-(3-{4-[4-(aminocarbonyl )benzyl]-1-piperidinyl}propyl)-N-(3,4-dichlorophenyl)-1-(methylsulfonyl)-4-piperidinecarboxamide (hereinafter to be referred to as compound B) (12 mg) with stirring and the mixture was thoroughly stirred to give a composition dispersed in a homogenous micro-state in the Vitamin E TPGS, which was then cooled to room temperature.

### Example 6

To Gelucire 44/14 (108 mg) melted at a temperature 20°C or more higher than the melting point was added compound B (12 mg) with stirring and the mixture was thoroughly stirred to give a composition dispersed in a homogenous micro-state in Gelucire 44/14.

### Example 7

To L/L/T (45/40/15) (108 mg) was added compound B (12 mg) with stirring and the mixture was thoroughly stirred to give a composition dispersed in a homogenous micro-state in L/L/T(45/40/15), which was then cooled to room temperature.

### Example 8

To Vitamin E TPGS (216 mg) melted at a temperature 20°C or more higher than the melting point was added N-(3,4-dichlorophenyl)-N-(3-{4-[4-(isopropylsulfonyl)benzyl]-1-piperidinyl}propyl)-1-(methylsulfonyl)-4-piperidinecarboxamide [hereinafter to be referred to as compound C] (24 mg) with stirring and the mixture was thoroughly stirred to allow complete dissolution in the Vitamin E TPGS, whereby a composition was obtained.

### Example 9

To L/L/T (45/40/15) (216 mg) was added compound C (24 mg) with stirring and the mixture was thoroughly stirred to allow complete dissolution in the L/L/T (45/40/15), whereby a composition was obtained.

### Example 10

To Vitamin E TPGS (216 mg) melted at a temperature 20°C or more higher than the melting point was added hydrochloride of compound C, N-(3,4-dichlorophenyl)-N-(3-{4-[4-(isopropylsulfonyl)benzyl]-1-piperidinyl}propyl)-1-(methylsulfonyl) -4-piperidinecarboxamide hydrochloride (hereinafter to be referred to as hydrochloride of compound C) (24 mg) with stirring and the mixture was thoroughly stirred to give a composition dispersed in a homogenous micro-state in the Vitamin E TPGS, which was then cooled to room temperature.

### Example 11

By adding hydrochloride (24 mg) of compound C to L/L/T (45/40/15) (216 mg) with stirring and the mixture was thoroughly stirred to give a composition dispersed in L/L/T (45/40/15) in a homogenous micro-state was obtained.

### Example 12

The compositions (520 mg each) prepared in Examples 2, 3, 8 and 10 were filled in gelatin capsules to give capsules.

### Example 13

The compositions (520 mg each) prepared in Examples 2, 3, 8 and 10 were filled in HPMC capsules to give capsules.

### Example 14

By adding 8 mL of purified water to Vitamin E TPGS (216 mg) and stirring the mixture for 30 min., an aqueous solution was obtained. To this aqueous solution was added compound C (24 mg) to give a clear emulsion composition.

### Example 15

To Gelucire 50/13 (135 mg) melted at a temperature 20°C or more higher than the melting point was added 1-acetyl-N-(3-{4-[4-(aminocarbonyl)benzyl]-1-piperidinyl}propyl)-N-(3-chloro-4-methylphenyl)-4-piperidinecarboxamide (hereinafter to be referred to as hydrochloride of compound D) (15 mg) with stirring and the mixture was thoroughly stirred to give a composition dispersed in a homogenous micro-state in Gelucire 50/13 was obtained, which was then cooled to room temperature.

### Example 16

By adding 15 mL of compound D to Vitamin E-TPGS (135 mg) melted at a temperature 20°C or more higher than the melting point and stirring the mixture, a composition dispersed in a homogenous micro-state in Vitamin E-TPGS was obtained, which was then cooled to room temperature.

### Example 17

By adding 15 mL of compound D to Labrasol (135 mg) heated to 70°C and thoroughly stirring the mixture to allow complete dissolution in Labrasol, a composition was obtained, which was then cooled to room temperature.

### Example 18

By adding 15 mL of compound D to L/L/T (45/40/15) (135 mg) heated to 70°C and thoroughly stirring the mixture to allow complete dissolution in L/L/T (45/40/15), a composition was obtained, which was then cooled to room temperature.

### Example 19

To Gelucire 50/13 (135 mg) melted at a temperature 20°C or more higher than the melting point was added N-(3-{4-[4-(aminocarbonyl)benzyl]-1-piperidinyl}propyl)-N-(3-chloro-4-methylphenyl)-1-(methylsulfonyl)-4-piperidinecarboxamide [hereinafter to be referred to as compound E] (15 mg) with stirring and the mixture was thoroughly stirred to give a composition dispersed in a homogenous micro-state in Gelucire 50/13, which was then cooled to room temperature.

### Example 20

To Vitamin E-TPGS (135 mg) melted at a temperature 20°C or more higher than the melting point was added compound E (15 mg) with stirring and the mixture was thoroughly stirred to give a composition dispersed in a homogenous micro-state in Vitamin E-TPGS, which was then cooled to room temperature.

### Example 21

By adding 15 mL of compound E to Labrasol (135 mg) heated to 70°C and thoroughly stirring the mixture, a composition dispersed in a homogenous micro-state in Labrasol was obtained, which was then cooled to room temperature.

### Example 22

By adding 15 mL of compound E to L/L/T (45/40/15) (135 mg) heated to 70°C and thoroughly stirring the mixture, a composition dispersed in a homogenous micro-state in L/L/T (45/40/15) was obtained, which was then cooled to room temperature.

### Example 23

By adding 15 mL of compound B to Labrasol (135 mg) heated to 70°C and thoroughly stirring the mixture, a composition dispersed in a homogenous micro-state in Labrasol was obtained, which was then cooled to room temperature.

### Example 24

By adding a pulverized mixture (30 mg) of compound D and an equivalent amount of sodium deoxycholate to L/L/T (45/40/15) (270 mg) heated to 70°C and thoroughly stirring the mixture to allow complete dissolution in L/L/T (45/40/15), whereby a composition was obtained, which was then cooled to room temperature.

### Example 25

To Vitamin E-TPGS (135 mg) melted at a temperature 20°C or more higher than the melting point was added a pulverized mixture (30 mg) of compound D and an equivalent amount of sodium deoxycholate with stirring and the mixture was thoroughly stirred to give a composition dispersed in a homogenous micro-state in the Vitamin E-TPGS, whereby a composition was obtained, which was then cooled to room temperature.

### Example 26

Compound D (20 mg) was added to oleic acid (80 mg) and the mixture was heated to 70°C and thoroughly mixed to allow complete dissolution in oleic acid. Thereto was added Gelucire 44/14 melted at 70°C and the mixture was thoroughly mixed and cooled to room temperature.

### Experimental Example 1

The composition prepared in Example 1 [hereinafter to be referred to as composition 1] was redissolved at 45°C and orally administered (dose 10 mg/kg) to rats (SD/IGS) using an oral sonde while feeding. At 0.5, 1, 2, 4 and 8 hrs. after the administration, blood was drawn from the caudal vein. The compound A concentration of the blood sample, which was obtained by centrifugal separation, was measured. As a control, compound A-containing 0.5% methyl cellulose suspension (compound A concentration 3 mg/mL) was orally administered (dose 10 mg/kg) in the same manner, and compound A concentration was measured.

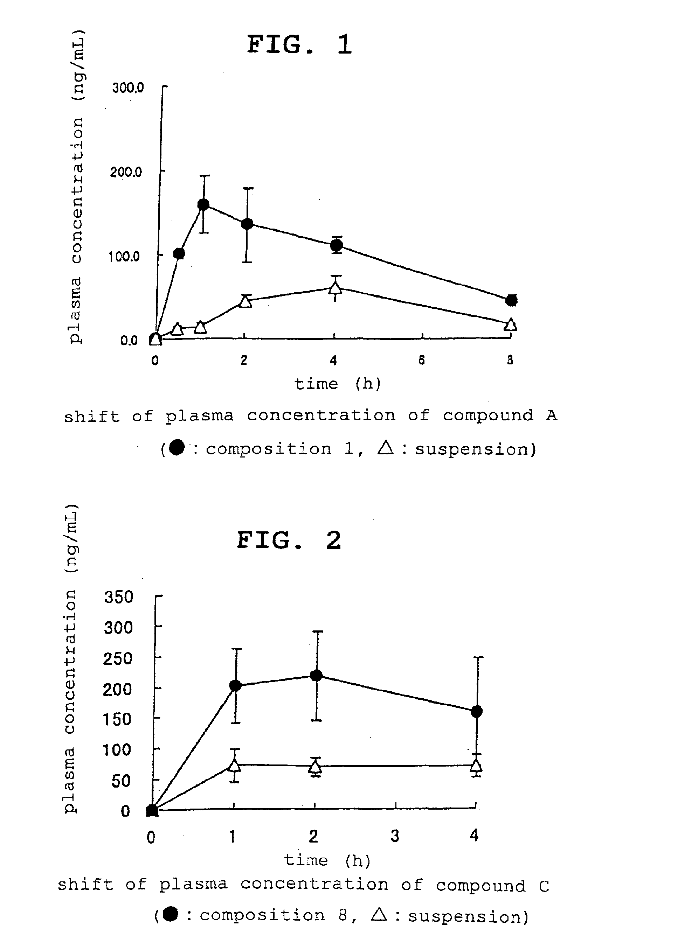

As a result, composition 1 drastically promoted absorption as compared to 0.5% methyl cellulose suspension, as shown in Fig. 1.

### Experimental Example 2

Purified water (1 mL) was added to the composition [hereinafter to be referred to as composition 8] (30 mg) prepared in Example 8 at this ratio to give a homogenous solution and orally administered (dose 10 mg/kg) to rats (SD/IGS) using an oral sonde with feeding. At 1, 2 and 4 hrs. after the administration, blood was drawn from the caudal vein. The compound C concentration of the blood sample, which was obtained by centrifugal separation, was measured. As a control, compound C hydrochloride-containing 0.5% methyl cellulose suspension (compound C concentration 3 mg/mL) was orally administered (dose 10 mg/kg) in the same manner, and compound C concentration was measured.

As a result, composition 8 drastically promoted absorption as compared to the suspension, as shown in Fig. 2.

### Industrial Applicability

The pharmaceutical composition for oral administration of the present invention is a preparation for oral administration having high bioavailability and shows extremely fine oral absorption. Therefore, it shows low inconsistency in the blood concentration of CCR5 antagonist and can be advantageously used for the prophylaxis or treatment of various HIV infectious diseases in human, such as AIDS.

This application is based on a patent application No. 2000-390870 filed in Japan, the contents of which are hereby incorporated by reference.

## Claims

1. An oral pharmaceutical composition comprising a compound having CCR5 antagonistic action, which is a compound represented by the formula 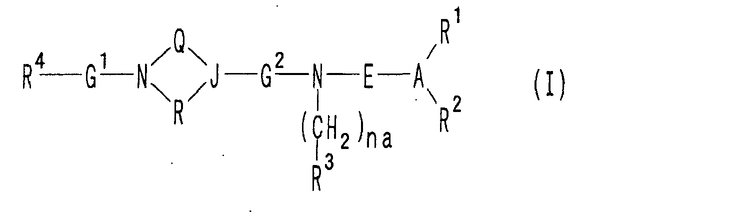 wherein
R¹ is a hydrogen atom, a hydrocarbon group optionally having substituent(s) or a nonaromatic heterocyclic group optionally having substituent(s),
R² is a hydrocarbon group optionally having substituent(s), a nonaromatic heterocyclic group optionally having substituent(s),
alternatively R¹ and R² may combine to form, together with A, a heterocyclic group optionally having substituent(s),
A is N or N⁺ -R5·Y⁻ (R⁵ is a hydrocarbon group and Y⁻ is a counter anion),
R³ is a cyclic hydrocarbon group optionally having substituent(s) or a heterocyclic group optionally having substituent(s),
na is 0 or 1,
R⁴ is a hydrogen atom, a hydrocarbon group optionally having substituent(s), a heterocyclic group optionally having substituent(s), an alkoxy group optionally having substituent(s), an aryloxy group optionally having substituent(s) or an amino group optionally having substituent(s),
E is a divalent chain hydrocarbon group optionally having substituent(s) other than oxo group,
G¹ is a bond, CO or SO₂,
G² is CO, SO₂ NHCO, CONH or OCO,
J is a methine or a nitrogen atom, and
Q and R are each a bond or a divalent C₁₋₃ chain hydrocarbon optionally having substituent(s),
provided that when G² is OCO, J is a methine, and neither Q nor R is a bond, and when G¹ is a bond, neither Q nor R is substituted by oxo group, or a salt thereof or a compound represented by the formula 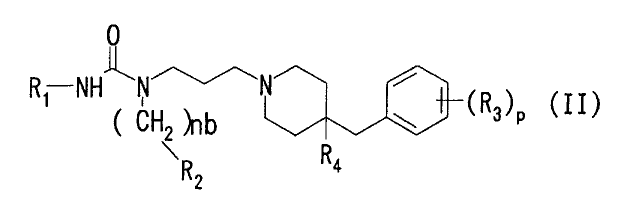 wherein
R₁ is a hydrocarbon group optionally having substituent(s),
R₂ is a cyclic hydrocarbon group optionally having substituent(s) or a heterocyclic group optionally having substituent(s),
R₃ is a halogen atom, a carbamoyl group optionally having substituent(s), a sulfamoyl group optionally having substituent(s), an acyl group derived from sulfonic acid, a C₁₋₄ alkyl group optionally having substituent(s), a C₁₋₄ alkoxy group optionally having substituent(s), an amino group optionally having substituent(s), a nitro group or a cyano group,
R₄ is a hydrogen atom or a hydroxy group,
nb is 0 or 1, and
p is 0 or an integer of 1 to 4, or a salt thereof,
which is dissolved or dispersed in at least one amphiphilic substance.

2. The composition of claim 1, wherein R¹ is a hydrogen atom, a hydrocarbon group selected from the following Group 2, which optionally has substituent(s) selected from the following Group 1, a 3- to 8-membered saturated or unsaturated nonaromatic heterocyclic group optionally having substituent(s) selected from the following Group 1, R² is a hydrocarbon group selected from the following Group 2, which optionally has substituent(s) selected from the following Group 1 or a 3- to 8-membered saturated or unsaturated nonaromatic heterocyclic group optionally having substituent(s) selected from the following Group 1, alternatively R¹ and R² may combine to form, together with A, a heterocyclic group selected from the following Group 4, which optionally has substituent(s) selected from the following Group 3, A is N or N⁺-R⁵·Y⁻ (Y⁻ is Cl⁻, Br⁻, I⁻, NO₃⁻, SO₄²⁻, PO₄³⁻ or CH₃SO₃⁻, and R⁵ is a hydrocarbon group selected from the following Group 2), R³ is a cyclic hydrocarbon group selected from the following Group 5, which optionally has substituent(s) selected from the following Group 1 or a heterocyclic group selected from the following Group 6, which optionally has substituent(s) selected from the following Group 1, R⁴ is a hydrogen atom, a hydrocarbon group selected from the following Group 2, which optionally has substituent(s) selected from the following Group 1, a heterocyclic group selected from the following Group 6, which optionally has substituent(s) selected from the following Group 1, a C₁₋₆ alkoxy group optionally having substituent(s) selected from the following Group 7, a C₆₋₁₄ aryloxy group optionally having substituent(s) selected from the following Group 8, an amino group optionally having substituent(s) selected from the following Group 9 or a cyclic amino group selected from the following Group 10, E is a divalent chain hydrocarbon group selected from the following Group 12, which optionally has substituent(s) other than oxo group selected from the following Group 12, Q and R are each a bond or a divalent C₁₋₃ chain hydrocarbon group selected from the following Group 13, which optionally has substituent(s) selected from the following Group 11, R₁ is a hydrocarbon group selected from the following Group 31, which optionally has substituent(s) selected from Group 29, R₂ is a cyclic hydrocarbon group selected from the following Group 35, which optionally has substituent(s) selected from Group 30 or a heterocyclic group selected from Group 32, which optionally has substituent(s) selected from Group 30, and R₃ is a halogen atom, a carbamoyl group, an N-monosubstituted carbamoyl group optionally having one selected from Group 19, an N,N-disubstituted carbamoyl group optionally having one selected from Group 19 and one selected from Group 20, a cyclic aminocarbonyl group selected from Group 21, sulfamoyl group, an N-monosubstituted sulfamoyl group optionally having one selected from Group 19, an N,N-disubstituted sulfamoyl group optionally having one selected from Group 19 and one selected from Group 20, a cyclic aminosulfonyl group selected from Group 36, an acyl group selected from Group 22, which is derived from sulfonic acid, a C₁₋₄ alkyl group optionally having substituent(s) selected from Group 30, a C₁₋₄ alkoxy group optionally having substituent(s) selected from Group 30, an amino group optionally having substituent(s) selected from Group 33, a cyclic amino group selected from Group 34, a nitro group or a cyano group, wherein in the above-mentioned,
Group 1 includes
(1) a C₁₋₆ alkyl group optionally substituted by a group selected from Group 14, (2) a C₂₋₆ alkenyl group optionally substituted by a group selected from Group 14, (3) a C₂₋₆ alkynyl group optionally substituted by a group selected from Group 14, (4) a C₆₋₁₄ aryl group optionally substituted by a group selected from Group 14, (5) a C₃₋₇ cycloalkyl group optionally substituted by a group selected from Group 14, (6) a C₃₋₆ cycloalkenyl group optionally substituted by a group selected from Group 14, (7) a heterocyclic group selected from Group 16, which is optionally substituted by a group selected from Group 15, (8) an amino group optionally having, as a substituent, C₁₋₆ alkylimidoyl, formylimidoyl, amidino or a group selected from Group 17, (9) a cyclic amino group selected from Group 10, (10) an imidoyl group optionally substituted by a group selected from Group 17, (11) an amidino group optionally substituted by a group selected from Group 17, (12) a hydroxy group optionally substituted by a group selected from Group 17, (13) a thiol group optionally substituted by a group selected from Group 17, (14) a carboxyl group, (15) a C₁₋₆ alkoxy-carbonyl group optionally substituted by a group selected from Group 18, (16) a C₇₋₂ aryloxy-carbonyl group optionally substituted by a group selected from Group 18, (17) a C₇₋₁₀ aralkyloxy-carbonyl group optionally substituted by a group selected from Group 18, (18) a carbamoyl group, (19) a monosubstituted carbamoyl group substituted by a group selected from Group 19, (20) a disubstituted carbamoyl group substituted by one selected from Group 19 and one selected from Group 20, (21) a cyclic aminocarbonyl group selected from Group 21, (22) a thiocarbamoyl group, (23) a monosubstituted thiocarbamoyl group substituted by a group selected from Group 19, (24) a disubstituted thiocarbamoyl group substituted by one selected from Group 19 and one selected from Group 20, (25) a sulfamoyl group, (26) an N-monosubstituted sulfamoyl group substituted by a group selected from Group 19, (27) an N,N-disubstituted sulfamoyl group substituted by one selected from Group 19 and one selected from Group 20, (28) a cyclic aminosulfonyl group selected from Group 22,(29) a halogen atom, (30) a cyano group, (31) a nitro group, (32) an acyl group selected from Group 22, which is derived from sulfonic acid, (33) a formyl group, (34) a C₂₋₆ alkanoyl, (35) a C₇₋₁₂ arylcarbonyl, (36) a C₁₋₆ alkylsulfinyl group optionally substituted by a group selected from Group 23 and (37) a C₆₋₁₄ arylsulfinyl group optionally substituted by a group selected from Group 23,
Group 2 includes
(1) a C₁₋₁₀ alkyl group, (2) a C₂₋₆ alkenyl group, (3) a C₂₋₆ alkynyl group, (4) a C₃₋₉ cycloalkyl group optionally condensed with benzene ring(s), (5) a C₃₋₆ cycloalkenyl group, (6) a C₄₋₆ cycloalkanedienyl group and (7) a C₆₋₁₄ aryl group,
Group 3 includes
(1) a hydroxy group, (2) a cyano group, (3) a nitro group, (4) an amino group, (5) an oxo group, (6) a halogen atom and (7) a group represented by the general formula -B¹R^{a} wherein R^{a} is a hydrocarbon group selected from Group 2 optionally having substituent(s) selected from Group 1, or a heterocyclic group selected from Group 6 optionally having substituent(s) selected from Group 1, and B¹ is a bond (single bond), -CR^{b}R^{c}-, -COO-, -CO-, -CR^{b}(OH)-, -CR^{b}R^{c}-S-, -CR^{b}R^{c}-SO₂-, -CO-NR^{b}-, -CS-NR^{b}-, -CO-S-,-CS-S-, -CO-NR^{b}-CO-NR^{c}-, -C(=NH)-NR^{b}-, -NR^{b}-, -NR^{b}-CO-, -NR^{b}-CS-, -NR^{b}-CO-NR^{c}-, -NR^{b}-CS-NR^{c}-, -NR^{b}-CO-O-, -NR^{b}-CS-O-, -NR^{b}-CO-S-, -NR^{b}-CS-S-, -NR^{b}-C(=NH)-NR^{c}, -NR^{b}-SO₂-, NR^{b}-NR^{c}-, -O-, -O-CO-, -O-CS-, -O-CO-O-, -O-CO-NR^{b}-, -O-C(=NH)-NR^{b}-, -S-, -SO-, -SO₂-, -SO₂-NR^{b}-, -S-CO-, -S-CS-, -S-CO-NR^{b}-, -S-CS-NR^{b}-and -S-C(=NH)-NR^{b}- (wherein R^{b} and R^{c} are each a hydrogen atom, a C₁₋₆ alkyl group optionally substituted by a group selected from Group 14, a C₂₋₆ alkenyl group optionally substituted by a group selected from Group 14, a C₂₋₆ alkynyl group optionally substituted by a group selected from Group 14, a C₆₋₁₄ aryl group optionally substituted by a group selected from Group 14, a C₃₋₇ cycloalkyl group optionally substituted by a group selected from Group 14, a C₃₋₆ cycloalkenyl group optionally substituted by a group selected from Group 14, a heterocyclic group selected from Group 6, which is optionally substituted by a group selected from Group 1, an acyl group selected from Group 22, which is derived from sulfonic acid, a C₁₋₆ alkanoyl or a C₇₋₁₂ arylcarbonyl group),
Group 4 includes
(1) a monocyclic heterocyclic group, (2) a fused heterocyclic ring wherein benzene is condensed and (3) a heterospirocyclic ring, which are rings optionally further containing, besides one nitrogen atom, nitrogen atom, oxygen atom, sulfur atom,
Group 5 includes
(1) a C₃₋₉ cycloalkyl optionally condensed with benzene ring(s), (2) a C₃₋₆ cycloalkenyl group, (3) a C₄₋₆ cycloalkanedienyl group and (4) a C₆₋₁₄ aryl group,
Group 6 includes
(1) a 5- or 6-membered aromatic monocyclic heterocyclic group selected from Group 24, (2) a 8- to 12-membered aromatic fused heterocycle group selected from Group 26 and (3) a 3- to 8-membered saturated or unsaturated nonaromatic heterocyclic group selected from Group 25 (aliphatic heterocyclic group), which heterocyclic groups containing, as a ring constituting atom (ring atom), 1 to 3 (at least 1) kinds of hetero atoms selected from oxygen atom, sulfur atom and nitrogen atom,
Group 7 includes
a C₃₋₆ cycloalkyl group optionally substituted by a group selected from Group 18, a C₆₋₁₀ aryl group optionally substituted by a group selected from Group 18, a C₇₋₁₀ aralkyl group optionally substituted by a group selected from Group 18 and a heterocyclic group selected from Group 16, which is optionally substituted by a group selected from Group 18,
Group 8 includes
a C₁₋₆ alkoxy group, a halogen atom, a C₁₋₆ alkyl group, an amino group, a hydroxy group, a cyano group and an amidino group,
Group 9 includes
(1) a C₁₋₆ alkyl group, (2) a C₁₋₆ alkanoyl, (3) benzoyl, (4) an optionally halogenated C₁₋₆ alkoxy-carbonyl, (5) a C₁₋₆ alkylimidoyl, (6) formylimidoyl and (7) amidino,
Group 10 includes
(1) 1-azetidinyl, (2) 1-pyrrolidinyl, (3) 1-piperidinyl, (4) 4-morpholinyl and (5) 1-piperazinyl optionally having substituent(s) selected from Group 27,
Group 11 includes
(1) a C₁₋₆ alkyl group optionally substituted by a group selected from Group 14, (2) a C₆₋₁₄ aryl group optionally substituted by a group selected from Group 14, (3) a C₃₋₇ cycloalkyl group optionally substituted by a group selected from Group 14, (4) a C₃₋₆ cycloalkenyl group optionally substituted by a group selected from Group 14, (5) a carboxyl group, (6) a C₁₋₆ alkoxy-carbonyl group optionally substituted by a group selected from Group 18, (7) a C₇₋₁₂ aryloxy-carbonyl group optionally substituted by a group selected from Group 18, (8) a C₇₋₁₀ aralkyloxy-carbonyl group optionally substituted by a group selected from Group 18, (9) a carbamoyl group, (10) a monosubstituted carbamoyl group substituted by a group selected from Group 19, (11) a disubstituted carbamoyl group substituted by one selected from Group 19 and one selected from Group 20, (12) a cyclic aminocarbonyl group selected from Group 21, (13) a thiocarbamoyl group, (14) a monosubstituted thiocarbamoyl group substituted by a group selected from Group 19, (15) a disubstituted thiocarbamoyl group substituted by one selected from Group 19 and one selected from Group 20, (16) an amino group optionally having, as a substituent, C₁₋₆ alkylimidoyl, formylimidoyl, amidino and a group selected from Group 17, (17) a cyclicamino group selected from Group 10, (18) a hydroxy group optionally substituted by a group selected from Group 17, (19) a thiol group optionally substituted by a group selected from Group 17, (20) C₁₋₆ alkanoyl, (21) C₇₋₁₂ arylcarbonyl, (22) an acyl group selected from Group 22, which is derived from sulfonic acid, (23) halogen, (24) nitro and (25) cyano,
Group 12 includes
a C₁₋₆ alkylene, a C₂₋₆ alkenylene and a C₂₋₆ alkynylene,
Group 13 includes
a C₁₋₃ alkylene, a C₂₋₃ alkenylene and a C₂₋₃ alkynylene,
Group 14 includes
(1) a C₁₋₆ alkoxy group optionally substituted by halogen, (2) a phenoxy optionally having halogen or carbamoyl as a substituent, (3) a halogen atom, (4) a C₁₋₆ alkyl group, (5) a halogen-substituted C₁₋₄ alkyl group, (6) a C₃₋₈ cycloalkyl, (7) an amino group, (8) an amino group having, as a substituent, 1 or 2 of carbamoyl, C₁₋₄ alkyl and C₁₋₄ alkylsulfonyl, (9) a carbamoyl group optionally substituted by C₁₋₆ alkyl, (10) a formyl, (11) a C₂₋₆ alkanoyl group, (12) a C₆₋₁₄ aryl group, (13) a C₆₋₁₄ arylcarbonyl, (14) a C₇₋₁₃ aralkylcarbonyl, (15) a hydroxy group, (16) a C₂₋₅ alkanoyloxy, (17) a C₇₋₁₃ aralkylcarbonyloxy, (18) a nitro group, (19) a sulfamoyl group, (20) an N-C₁₋₄ alkylsulfamoyl, (21) phenylthio, (22) a C₁₋₄ alkylphenylthio, (23) -N=N-phenyl, (24) a cyano group, (25) an oxo group, (26) an amidino group, (27) a carboxyl group, (28) a C₁₋₄ alkoxy-carbonyl, (29) a C₁₋₆ alkylthio, (30) a C₁₋₆ alkylsulfinyl, (31) a C₁₋₆ alkylsulfonyl, (32) a C₆₋₁₄ arylthio, (33) a C₆₋₁₄ arylsulfinyl, (34) a C₆₋₁₄ arylsulfonyl and (35) a heterocyclic group selected from Group 6,
Group 15 includes
a C₁₋₆ alkyl group, a C₁₋₆ alkanoyl, a C₇₋₁₃ arylcarbonyl, a C₁₋₆ alkylsulfonyl, aminosulfonyl, a mono-C₁₋₆ alkylaminosulfonyl, a di-C₁₋₆ alkylaminosulfonyl and a C₁₋₄ alkyl halide,
Group 16 includes
(1) an aromatic heterocyclic group selected from Group 24 and Group 26 and (2) a saturated or unsaturated nonaromatic heterocyclic group selected from Group 25, which heterocyclic groups containing, as a ring constituting atom (ring atom), 1 to 3 (at least 1) kinds of hetero atoms selected from oxygen atom, sulfur atom and nitrogen atom,
Group 17 includes
(1) a C₁₋₆ alkyl group optionally having halogen or C₁₋₆ alkoxy as a substituent, (2) a c₆₋₁₂ aryl group, (3) a C₁₋₄ alkyl-substituted C₆₋₁₂ aryl group, (4) a C₃₋₈ cycloalkyl group optionally having halogen or C₁₋₆ alkoxy as a substituent, (5) a C₁₋₆ alkoxy group, (6) a C₁₋₆ alkanoyl, (7) a C₇₋₁₃ arylcarbonyl, (8) a C₁₋₄ alkyl-substituted C₇₋₁₃ arylcarbonyl, (9) a C₁₋₆ alkylsulfonyl, (10) a C₆₋₁₄ arylsulfonyl, (11) aminosulfonyl, (12) a substituted aminosulfonyl mono- or di-substituted by C₁₋₄ alkyl and (13) an optionally halogenated C₁₋ 6 alkoxy-carbonyl,
Group 18 includes
(1) a hydroxy group, (2) an amino group, (3) an amino group mono- or di-substituted by a group selected from Group 28, (4) a halogen atom, (5) a nitro group, (6) a cyano group, (7) a C₁₋ 6 alkyl group optionally-substituted by halogen atom and (8) a C₁₋₆ alkoxy group optionally substituted by halogen atom,
Group 19 includes
a C₁₋₆ alkyl group optionally substituted by a group selected from Group 18, a C₃₋₆ cycloalkyl group optionally substituted by a group selected from Group 18, a C₆₋₁₀ aryl group optionally substituted by a group selected from Group 18, a C₇₋₁₀ aralkyl group optionally substituted by a group selected from Group 18, a C₁₋₆ alkoxy group optionally substituted by a group selected from Group 18 and a heterocyclic group selected from Group 16, which is optionally substituted by a group selected from Group 18,
Group 20 includes
a C₁₋₆ alkyl group, a C₃₋₆ cycloalkyl group and a C₇₋₁₀ aralkyl group,
Group 21 includes
1-azetidinylcarbonyl, 1-pyrrolidinylcarbonyl, 1-piperidinylcarbonyl, 4-morpholinylcarbonyl and 1-piperazinylcarbonyl optionally substituted by a group selected from Group 27,
Group 22 includes
a C₁₋₁₀ alkylsulfonyl optionally having substituent(s) selected from Group 18, a C₂₋₆ alkenylsulfonyl optionally having substituent(s) selected from Group 18, a C₂₋₆ alkynylsulfonyl optionally having substituent(s) selected from Group 18, a C₃₋₉ cycloalkylsulfonyl optionally having substituent(s) selected from Group 18, a C₃₋₉ cycloalkenylsulfonyl optionally having substituent(s) selected from Group 18, a C₆₋₁₄ arylsulfonyl optionally having substituent(s) selected from Group 18 and a C₇₋₁₀ aralkylsulfonyl optionally having substituent(s) selected from Group 18,
Group 23 includes
a C₁₋₆ alkoxy group, a halogen atom, a C₁₋₆ alkyl group, an amino group, a hydroxy group, a cyano group and an amidino group,
Group 24 includes
furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl,
Group 25 includes
oxyranyl, azetidinyl, oxetanyl, thietanyl, pyrrolidinyl, tetrahydrofuryl, thiolanyl, piperidinyl, tetrahydropyranyl, morpholinyl, thiomorpholinyl and piperazinyl,
Group 26 includes
benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, benzodioxolyl, benzimidazolyl, 2,1,1-benzoxadiazolyl, 1H-benzotriazolyl, quinolyl, isoquinolyl, cinnolinyl, quinazolynyl, quinoxalinyl, phthalazinyl, naphthylidinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-ajpyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl,
Group 27 includes
a C₁₋₆ alkyl group, a C₇₋₁₀ aralkyl group and a C₆₋₁₀ aryl group, Group 28 includes
a C₁₋₆ alkyl group, a C₁₋₆ alkanoyl, a C₇₋₁₃ arylcarbonyl and a C₁₋₆ alkylsulfonyl,
Group 29 includes
1) a hydrocarbon group selected from Group 31, which optionally has substituent(s) selected from Group 30, 2) a heterocyclic group selected from Group 32, which optionally has substituent(s) selected from Group 30, 3) a C₁₋₄ alkoxy group optionally having substituent(s) selected from Group 30, 4) a C₁₋₄ alkylthio group optionally having substituent(s) selected from Group 30, 5) a C₂₋₆ alkoxycarbonyl group optionally having substituent(s) selected from Group 30, 6) a C₁₋₆ alkanoyl group optionally having substituents selected from Group 30, 7) an amino group optionally having substituent(s) selected from Group 33, 8) a cyclic amino group selected from Group 34, 9) a halogen atom, 10) a nitro group, 11) a cyano group, 12) a carbamoyl group, 13) a monosubstituted carbamoyl group substituted by a group selected from Group 19, 14) a disubstituted carbamoyl group substituted by one selected from Group 19 and one selected from Group 20, 15) a cyclic aminocarbonyl group selected from Group 21, 16) a sulfamoyl group, 17) an N-monosubstituted sulfamoyl group substituted by a group selected from Group 19, 18) an N,N-disubstituted sulfamoyl group substituted by one selected from Group 19 and one selected from Group 20, 19) an acyl group selected from Group 22, which is derived from sulfonic acid,
Group 30 includes
1) a C₁₋₆ alkoxy group, 2) a halogen atom, 3) a C₁₋₆ alkyl group, 4) a C₁₋₄ alkynyl group, 5) an amino group, 6) a hydroxy group, 7) a cyano group and 8) an amidino group,
Group 31 includes
1) a C₁₋₆ alkyl group, 2) a C₃₋₈ cycloalkyl group and 3) a C₆₋₁₄ aryl group,
Group 32 includes
1) an aromatic monocyclic heterocyclic group selected from Group 24, 2) an aromatic fused heterocyclic group selected from Group 26 and 3) a saturated or unsaturated nonaromatic heterocyclic group selected from Group 25,
Group 33 includes
1) a C₁₋₆ alkyl, 2) a C₁₋₆ alkanoyl, 3) a C₇₋₁₃ arylcarbonyl, 4) an optionally halogenated C₂₋₆ alkoxycarbonyl, 5) a C₁₋₆ alkylimidoyl, 6) a formylimidoyl and 7) an amidino,
Group 34 includes
1) 1-azetidinyl, 2) 1-pyrrolidinyl, 3) 1-piperidinyl, 4) 4-morpholinyl, 5) 1-piperazinyl and 6) a 1-piperazinyl optionally having C₁₋₆ alkyl, C₇₋₁₀ aralkyl or C₆₋₁₀ aryl at the 4-position,
Group 35 includes
a C₃₋₉ cycloalkyl, 1-indanyl, 2-indanyl, a C₃₋₆ cycloalkenyl, a C₄₋₆ cycloalkanedienyl and a C₆₋₁₄ aryl,
Group 36 includes
1-azetidinylsulfonyl, 1-pyrrolidinylsulfonyl, 1-piperidinylsulfonyl, 4-morpholinylsulfonyl and a 1-piperazinylsulfonyl optionally substituted by a group selected from Group 27.

3. The composition of claim 1, wherein the compound having CCR5 antagonistic action of claim 1 is dissolved in at least one amphiphilic substance.

4. The composition of claim 1, wherein the amphiphilic substance contains at least one solubilizing agent.

5. The composition of claim 1, wherein the amphiphilic substance contains at least one kind of lipid.

6. The composition of claim 1, which is liquid or semi-solid at about 15°C to about 25°C.

7. The composition of claim 1, which is a solid at about 15°C to about 25°C.

8. The composition of claim 1, which has a self-emulsifying action.

9. The composition of claim 1, wherein the amphiphilic substance comprises a combination of one or more kinds selected from water-soluble vitamin E derivatives, saturated polyglycolated glycerides, unsaturated polyglycolated glycerides, sorbitan fatty acid esters, polyglycerine fatty acid ester, polyoxyethylene polypropylene glycols, polyoxyethylene castor oil derivatives and propylene glycol laurates.

10. The composition of claim 1, wherein the amphiphilic substance comprises a combination of one or more kinds selected from water-soluble vitamin E derivatives, saturated polyglycolated glycerides, unsaturated polyglycolated glycerides.

11. The composition of claim 1, comprising a water-soluble vitamin E derivative as an amphiphilic substance.

12. The composition of claim 11, wherein the water-soluble vitamin E derivative is D-α-tocopherol polyethylene glycol 1000 succinate.

13. The composition of claim 1, wherein the emulsion is formed by adding water to a system wherein a compound having CCR5 antagonistic action is dissolved or dispersed in at least one amphiphilic substance.

14. The composition of claim 1, wherein the compound having CCR5 antagonistic action is N-(3,4-dichlorophenyl)-1-(methylsulfonyl)-N-{3-[4-({4-[(methylsulfonyl)amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl) -4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-( 3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide or a salt thereof.

15. An oral pharmaceutical composition wherein a piperidine compound having CCR5 antagonistic action is dissolved or dispersed in at least one amphiphilic substance.

16. The composition of claim 1, comprising the piperidine compound having CCR5 antagonistic action of claim 1, a protease inhibitor and/or a reverse transcriptase inhibitor in combination.

17. A capsule containing the composition of claim 1.

18. The composition of claim 1, comprising water.

19. A process for preparing an oral pharmaceutical composition, which comprises preparing the same from an aqueous dispersion or aqueous solution of a piperidine compound having CCR5 antagonistic action and at least one amphiphilic substance.

20. The process of claim 19, comprising adding a piperidine compound having CCR5 antagonistic action to an aqueous solution containing at least one amphiphilic substance and stirring.

21. A process for preparing an oral pharmaceutical composition, which comprises dissolving or dispersing a piperidine compound having CCR5 antagonistic action in an amphiphilic substance melted at a temperature higher than the melting point of the amphiphilic substance in a homogenous micro-state, followed by cooling.

22. The composition of claim 1, which is a prophylactic or therapeutic agent of an HIV infectious disease.

23. The composition of claim 1, which is a prophylactic or therapeutic agent of AIDS.

24. A method of prophylaxis or treatment of HIV infection, which comprises administration of the oral pharmaceutical composition of claim 1 to a mammal.

25. Use of at least one kind of amphiphilic substance for the production of an oral pharmaceutical composition comprising a compound having CCR5 antagonistic action, which is a compound represented by the formula 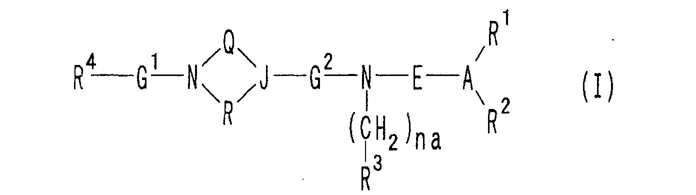 wherein
R¹ is a hydrogen atom, a hydrocarbon group optionally having substituent(s) or a nonaromatic heterocyclic group optionally having substituent(s),
R² is a hydrocarbon group optionally having substituent(s), a nonaromatic heterocyclic group optionally having substituent(s),
alternatively R¹ and R² may combine to form, together with A, a heterocyclic group optionally having substituent(s),
A is N or N+-R⁵·Y⁻ (R⁵ is a hydrocarbon group and Y⁻ is a counter anion),
R³ is a cyclic hydrocarbon group optionally having substituent(s) or a heterocyclic group optionally having substituent(s),
na is 0 or 1,
R⁴ is a hydrogen atom, a hydrocarbon group optionally having substituent(s), a heterocyclic group optionally having substituent(s), an alkoxy group optionally having substituent(s), an aryloxy group optionally having substituent(s) or an amino group optionally having substituent(s),
E is a divalent chain hydrocarbon group optionally having substituent(s) other than oxo group,
G¹ is a bond, CO or SO₂,
G² is CO, SO₂, NHCO, CONH or OCO,
J is a methine or a nitrogen atom, and
Q and R are each a bond or a divalent C₁₋₃ chain hydrocarbon optionally having substituent(s),
provided that when G² is OCO, J is a methine, and neither Q nor R is a bond, and when G¹ is a bond, neither Q nor R is substituted by oxo group,
or a salt thereof or a compound represented by the formula 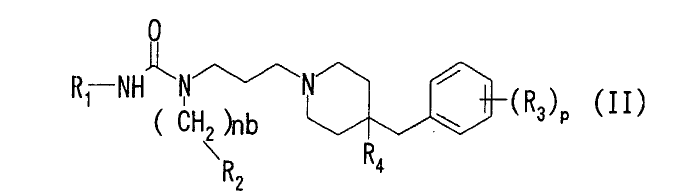 wherein
R₁ is a hydrocarbon group optionally having substituent(s),
R₂ is a cyclic hydrocarbon group optionally having substituent(s) or a heterocyclic group optionally having substituent(s),
R₃ is a halogen atom, a carbamoyl group optionally having substituent(s), a sulfamoyl group optionally having substituent(s), an acyl group derived from sulfonic acid, a C₁₋₄ alkyl group optionally having substituent(s), a C₁₋₄ alkoxy group optionally having substituent(s), an amino group optionally having substituent(s), a nitro group or a cyano group,
R₄ is a hydrogen atom or a hydroxy group,
nb is 0 or 1, and
p is 0 or an integer of 1 to 4, or a salt thereof.

26. A commercial package comprising the composition of the claim 22 or 23 and written matter associated therewith, the written matter stating that the composition can or should be used for the prophylaxis or treatment of an HIV infectious disease or AIDS.
